# EUROPEAN PATENT APPLICATION

(11) **EP 4 847 613 A1**
(43) Date of publication of application: **02.12.2026**
(21) Application number: 25744732.6
(22) Date of filing: 24.01.2025
(51) Int. Cl.: C12N 15/113, A61K 31/7125, A61K 31/713, A61K 31/712, A61K 48/00

(54) **USES OF DOUBLE-STRANDED SIRNA AND CONJUGATE THEREOF**

(30) Priority: 26.01.2024 CN 202410115401; 16.04.2024 CN 202410463647; 26.04.2024 CN 202410518440; 13.09.2024 CN 202411287605; 17.01.2025 CN 202510084561
(71) Applicant: CMS Research & Development Pte. Ltd., Singapore 179803 (SG)
(72) Inventor: CHEN, Shi, Shanghai 200131 (CN); HU, Yanbin, Shanghai 200131 (CN); CHEN, Sijin, Shanghai 200131 (CN); HE, Haiying, Shanghai 200131 (CN); CHEN, Shuhui, Shanghai 200131 (CN)
(74) Representative: Haseltine Lake Kempner LLP
(86) International application number: PCT/CN2025/074742
(87) International publication number: WO 2025/157269

(57) **Abstract**

A double-stranded siRNA and a use thereof. Specifically disclosed are uses of a double-stranded siRNA containing non-natural nucleotides and a conjugate of the double-stranded siRNA, targeting an inhibin βE gene, and being capable of being applied to treatment of metabolic disorder-related diseases.

## Description

The present application claims priority to Chinese patent application No. 2024101154015 filed on January 26, 2024, Chinese patent application No. 2024104636471 filed on April 16, 2024, Chinese patent application No. 202410518440X filed on April 26, 2024, Chinese patent application No. 2024112876053 filed on September 13, 2024, and Chinese patent application No. 2025100845612 filed on January 17, 2025. The contents of the above Chinese patent applications are incorporated herein by reference in their entirety.

### TECHNICAL FIELD

The present disclosure relates to double-stranded siRNAs and the use thereof, specifically to the use of a class of double-stranded siRNAs containing non-natural nucleotides and conjugates thereof.

### BACKGROUND

Abdominal obesity, also known as central obesity, is a form of obesity characterized by the accumulation of adipose tissue around the visceral organs, mesentery, and aorta within the abdominal cavity. It is primarily manifested by an increase in waist circumference. According to the Chinese occupational health standards, abdominal obesity is diagnosed when the waist circumference exceeds 90 cm in men or 85 cm in women.

Abdominal obesity is highly prevalent in China. A survey conducted between 2012 and 2015 showed that the prevalence of abdominal obesity among the Chinese population was 29.1%, including 28.6% in men and 29.6% in women. Based on the prevalence, the number of adults with abdominal obesity nationwide is estimated to be approximately 277.8 million.

Abdominal obesity is one of the manifestations of metabolic syndrome and is closely associated with type 2 diabetes and coronary heart disease. A health survey involving more than 310,000 Korean individuals showed that general obesity alone was not associated with an increased risk of major adverse cardiovascular events. In contrast, individuals with abdominal obesity alone or with both general obesity and abdominal obesity exhibited a significantly increased risk of major adverse cardiovascular events.

However, no drug has been approved for the treatment of abdominal obesity, indicating an unmet clinical need in the art.

The Inhibin E (INHBE) gene encodes the inhibin βE subunit, which is a component of activins/inhibins belonging to the TGF-β superfamily. Studies on individuals carrying loss-of-function mutations in the INHBE gene have demonstrated that INHBE expression is closely associated with abdominal fat accumulation and fat distribution. Individuals carrying loss-of-function mutations in the INHBE gene exhibit a lower waist-to-hip ratio adjusted for BMI (WHRadjBMI), a commonly used indicator for assessing abdominal obesity. In addition, individuals carrying loss-of-function mutations in the INHBE gene also tend to have a reduced incidence of coronary heart disease and type 2 diabetes. More importantly, individuals carrying INHBE mutations that result in approximately 50% reduction in INHBE expression exhibit lower triglyceride levels and higher high-density lipoprotein levels, thereby displaying an overall healthier metabolic phenotype.

The mechanism of RNA interference (RNAi or siRNA) has attracted extensive attention since its discovery in 1998. RNA interference regulates the function of target genes by reducing the level of corresponding target mRNAs, thereby achieving the desired pharmacological effects. In recent years, several RNAi therapeutics have been approved for marketing, demonstrating their feasibility in humans. Drugs that exert their pharmacological effects through the mechanism are referred to as RNAi therapeutics.

### SUMMARY

The present disclosure provides a double-stranded siRNA or a pharmaceutically acceptable salt thereof, wherein the double-stranded siRNA comprises a sense strand and an antisense strand capable of forming a duplex region; the sense strand comprises at least 16 consecutive nucleotides from any one of the sequences set forth in SEQ ID NOs: 1-80 and SEQ ID NOs: 161-188; the antisense strand comprises at least 16 consecutive nucleotides from any one of the sequences set forth in SEQ ID NOs: 81-160 and SEQ ID NOs: 189-230;
the nucleotides comprised in the sense strand and the nucleotides comprised in the antisense strand are modified or unmodified nucleotides.

In some embodiments of the present disclosure, in the double-stranded siRNA or a pharmaceutically acceptable salt thereof, the sense strand comprises the nucleotides at positions 1-17, 1-18, 1-19, 1-20, 1-21, 2-17, 2-18, 2-19, 2-20, 2-21, 3-18, 3-19, 3-20, 3-21, 4-19, 4-20, 4-21, or 5-21 from the 5' end of any one of the sequences set forth in SEQ ID NOs: 1-80 and SEQ ID NOs: 161-188; preferably, the sense strand comprises the nucleotides at positions 1-17, 1-18, 1-19, 2-17, 2-18, 2-19, 2-20, 3-18, 3-19, 3-20, 4-19, 4-20, 4-21, or 5-21 from the 5' end of any one of the sequences set forth in SEQ ID NOs: 1-80 and SEQ ID NOs: 161-188.

In some embodiments of the present disclosure, in the double-stranded siRNA or a pharmaceutically acceptable salt thereof, the antisense strand comprises the nucleotides at positions 1-17, 1-18, 1-19, 1-20, 1-21, 1-22, 1-23, 2-18, 2-19, 2-20, 2-21, 2-22, 2-23, 3-19, 3-20, 3-21, 3-22, 3-23, 4-20, 4-21, 4-22, 4-23, 5-21, 5-22, 5-23, 6-22, 6-23, or 7-23 from the 5' end of any one of the sequences set forth in SEQ ID NOs: 81-160 and SEQ ID NOs: 189-230; preferably, the antisense strand comprises the nucleotides at positions 1-19, 1-20, 1-21, 1-22, 1-23, 2-18, 2-19, 2-20, 2-21, 3-20, or 3-21 from the 5' end of any one of the sequences set forth in SEQ ID NOs: 81-160 and SEQ ID NOs: 189-230.

In some embodiments of the present disclosure, in the double-stranded siRNA or a pharmaceutically acceptable salt thereof, at least one nucleotide residue on the sense strand, the antisense strand, or both the sense strand and the antisense strand is independently and optionally replaced by dR, R, or Z, wherein the dR is 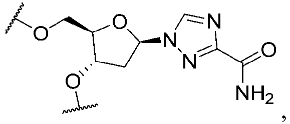 the R is 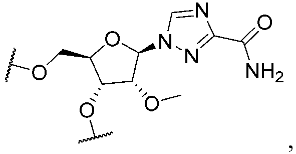 and the Z is

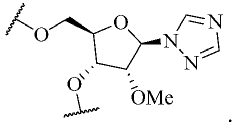

In some embodiments of the present disclosure, in the double-stranded siRNA or a pharmaceutically acceptable salt thereof, 1 to 4 nucleotide residues at positions 2, 5-7, 12, and 14-18 from the 5' end of the antisense strand are independently and optionally replaced by dR, R, or Z.

In some embodiments of the present disclosure, in the double-stranded siRNA or a pharmaceutically acceptable salt thereof, 1 to 4 nucleotides at positions 2, 5-7, 12, and 14-18 from the 5' end of the antisense strand are independently and optionally 2'-deoxynucleotides.

In some embodiments of the present disclosure, in the double-stranded siRNA or a pharmaceutically acceptable salt thereof, the first nucleoside at the 5' end of the antisense strand may be replaced by a vinylphosphonate-containing nucleoside analog.

In some embodiments of the present disclosure, in the double-stranded siRNA or a pharmaceutically acceptable salt thereof, the vinylphosphonate-containing nucleoside analog is selected from the following structural moieties:

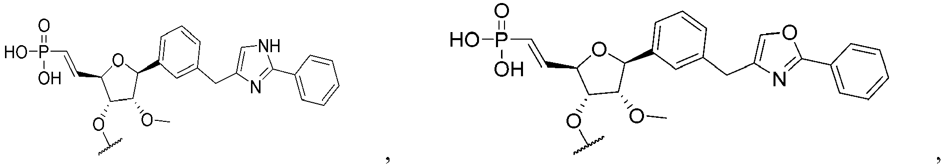

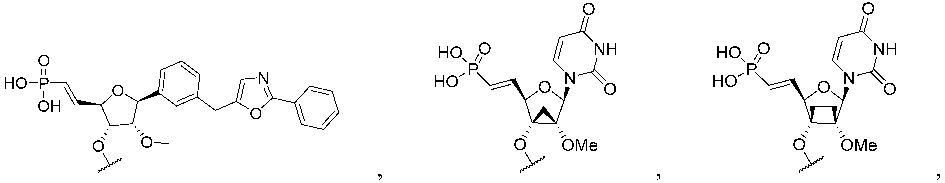

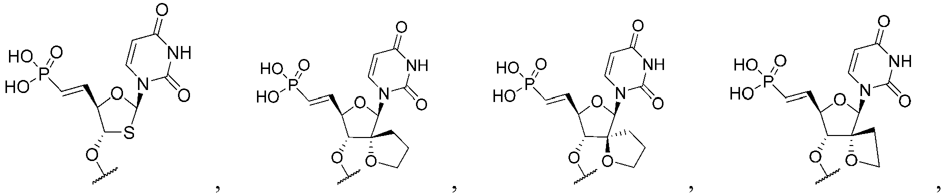

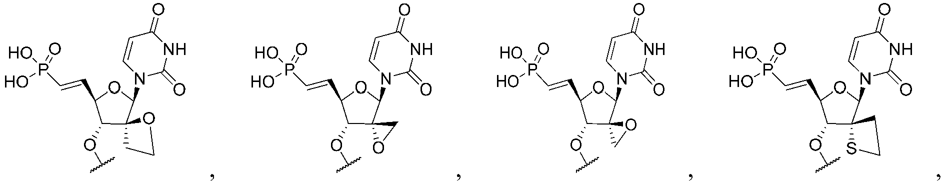

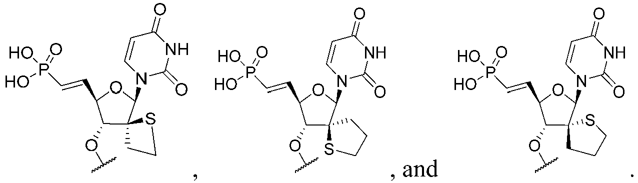

The present disclosure provides a double-stranded siRNA or a pharmaceutically acceptable salt thereof, comprising a sense strand and an antisense strand capable of forming a duplex region, wherein the sense strand comprises the nucleotides at positions 1-17, 1-18, 1-19, 1-20, 1-21, 2-17, 2-18, 2-19, 2-20, 2-21, 3-18, 3-19, 3-20, 3-21, 4-19, 4-20, 4-21, or 5-21 from the 5' end of any one of the sequences set forth in SEQ ID NOs: 1-80, wherein 1 to 4 nucleotide residues on the sense strand are independently and optionally replaced by dR, R, or Z, wherein the dR is 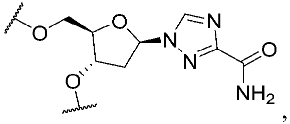 the R is 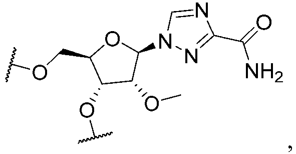 and the Z is 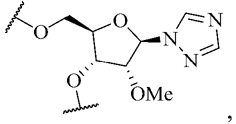 and wherein the nucleotides on the sense strand are optionally modified.

The present disclosure provides a double-stranded siRNA or a pharmaceutically acceptable salt thereof, comprising a sense strand and an antisense strand capable of forming a duplex region, wherein the antisense strand comprises the nucleotides at positions 1-17, 1-18, 1-19, 1-20, 1-21, 1-22, 1-23, 2-18, 2-19, 2-20, 2-21, 2-22, 2-23, 3-19, 3-20, 3-21, 3-22, 3-23, 4-20, 4-21, 4-22, 4-23, 5-21, 5-22, 5-23, 6-22, 6-23, or 7-23 from the 5' end of any one of the sequences set forth in SEQ ID NOs: 81-160, wherein 1 to 4 nucleotide residues on the antisense strand are independently and optionally replaced by dR, R, or Z, wherein the dR is 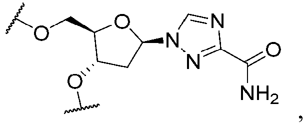 the R is 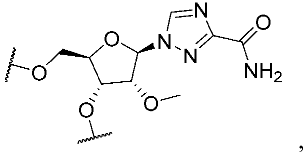 and the Z is 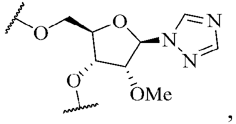 and wherein the nucleotides on the antisense strand are optionally modified.

The present disclosure provides a double-stranded siRNA or a pharmaceutically acceptable salt thereof, comprising a sense strand and an antisense strand capable of forming a duplex region, wherein the sense strand comprises the nucleotides at positions 1-17, 1-18, 1-19, 1-20, 1-21, 2-17, 2-18, 2-19, 2-20, 2-21, 3-18, 3-19, 3-20, 3-21, 4-19, 4-20, 4-21, or 5-21 from the 5' end of any one of the sequences set forth in SEQ ID NOs: 161-188, wherein 1 to 4 nucleotide residues on the sense strand are independently and optionally replaced by dR, R, or Z, wherein the dR is 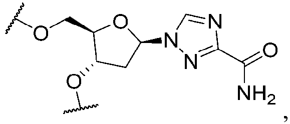 the R is 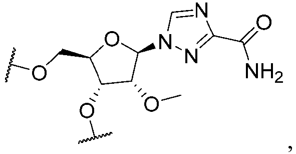 and the Z is 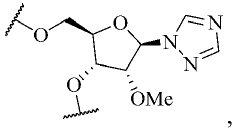 and wherein the nucleotides on the sense strand are optionally modified.

The present disclosure provides a double-stranded siRNA or a pharmaceutically acceptable salt thereof, comprising a sense strand and an antisense strand capable of forming a duplex region, wherein the antisense strand comprises the nucleotides at positions 1-17, 1-18, 1-19, 1-20, 1-21, 1-22, 1-23, 2-18, 2-19, 2-20, 2-21, 2-22, 2-23, 3-19, 3-20, 3-21, 3-22, 3-23, 4-20, 4-21, 4-22, 4-23, 5-21, 5-22, 5-23, 6-22, 6-23, or 7-23 from the 5' end of any one of the sequences set forth in SEQ ID NOs: 189-230, wherein 1 to 4 nucleotide residues on the antisense strand are independently and optionally replaced by dR, R, or Z, wherein the dR is 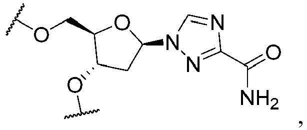 the R is 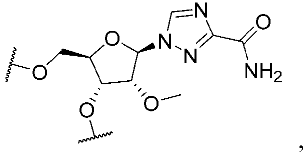 and the Z is 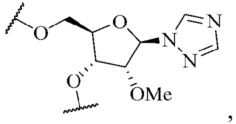 and wherein the nucleotides on the antisense strand are optionally modified.

The present disclosure provides a double-stranded siRNA or a pharmaceutically acceptable salt thereof, comprising a sense strand and an antisense strand capable of forming a duplex region, wherein the sense strand comprises the nucleotides at positions 1-17, 1-18, 1-19, 1-20, 1-21, 2-17, 2-18, 2-19, 2-20, 2-21, 3-18, 3-19, 3-20, 3-21, 4-19, 4-20, 4-21, or 5-21 from the 5' end of any one of the sequences set forth in SEQ ID NOs: 161-188, wherein 1 to 4 nucleotide residues on the sense strand are independently and optionally replaced by dR, R, or Z, wherein the dR is 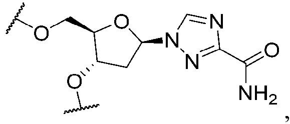 the R is 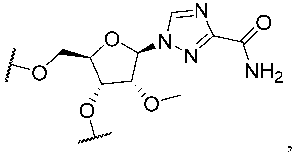 and the Z is

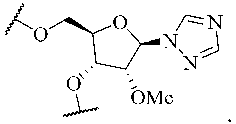

The present disclosure provides a double-stranded siRNA or a pharmaceutically acceptable salt thereof, comprising a sense strand and an antisense strand capable of forming a duplex region, wherein the antisense strand comprises the nucleotides at positions 1-17, 1-18, 1-19, 1-20, 1-21, 1-22, 1-23, 2-18, 2-19, 2-20, 2-21, 2-22, 2-23, 3-19, 3-20, 3-21, 3-22, 3-23, 4-20, 4-21, 4-22, 4-23, 5-21, 5-22, 5-23, 6-22, 6-23, or 7-23 from the 5' end of any one of the sequences set forth in SEQ ID NOs: 189-230, wherein 1 to 4 nucleotide residues on the antisense strand are independently and optionally replaced by dR, R, or Z, wherein the dR is 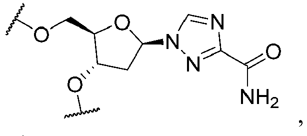 the R is 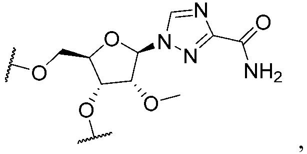 and the Z is

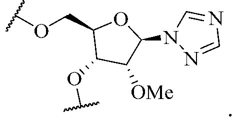

In some embodiments of the present disclosure, the sense strand comprises the nucleotides at positions 1-17, 1-18, 1-19, 2-17, 2-18, 2-19, 2-20, 3-18, 3-19, 3-20, 4-19, 4-20, 4-21, or 5-21 from the 5' end of any one of the sequences set forth in SEQ ID NOs: 1-80, wherein 1 to 4 nucleotide residues on the sense strand are independently and optionally replaced by dR, R, or Z, wherein the dR is 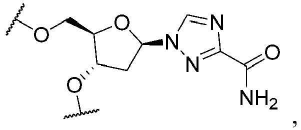 the R is 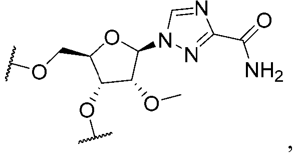 and the Z is 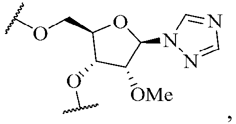 and wherein the nucleotides on the sense strand comprise at least one modified nucleotide.

In some embodiments of the present disclosure, the antisense strand comprises the nucleotides at positions 1-19, 1-20, 1-21, 1-22, 1-23, 2-18, 2-19, 2-20, 2-21, 3-20, or 3-21 from the 5' end of any one of the sequences set forth in SEQ ID NOs: 81-160, wherein 1 to 4 nucleotide residues on the antisense strand are independently and optionally replaced by dR, R, or Z, wherein the dR is 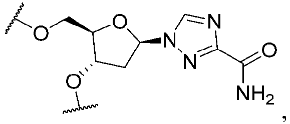 the R is 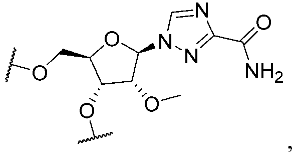 and the Z is 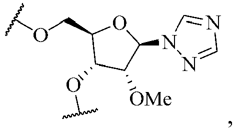 and wherein the nucleotides on the antisense strand comprise at least one modified nucleotide.

In some embodiments of the present disclosure, the sense strand comprises the nucleotides at positions 1-17, 1-18, 1-19, 2-17, 2-18, 2-19, 2-20, 3-18, 3-19, 3-20, 4-19, 4-20, 4-21, or 5-21 from the 5' end of any one of the sequences set forth in SEQ ID NOs: 161-188, wherein 1 to 4 nucleotide residues on the sense strand are independently and optionally replaced by dR, R, or Z.

In some embodiments of the present disclosure, the antisense strand comprises the nucleotides at positions 1-19, 1-20, 1-21, 1-22, 1-23, 2-18, 2-19, 2-20, 2-21, 3-20, or 3-21 from the 5' end of any one of the sequences set forth in SEQ ID NOs: 189-230, wherein 1 to 4 nucleotide residues on the antisense strand are independently and optionally replaced by dR, R, or Z.

In some embodiments of the present disclosure, the sense strand comprises the nucleotides at positions 1-17, 1-18, 1-19, 2-17, 2-18, 2-19, 2-20, 3-18, 3-19, 3-20, 4-19, 4-20, 4-21, or 5-21 from the 5' end of any one of the sequences set forth in SEQ ID NOs: 1-80.

In some embodiments of the present disclosure, the sense strand comprises the nucleotides at positions 1-17, 1-18, 1-19, 2-17, 2-18, 2-19, 2-20, 3-18, 3-19, 3-20, 4-19, 4-20, 4-21, or 5-21 from the 5' end of any one of the sequences set forth in SEQ ID NOs: 161-188.

In some embodiments of the present disclosure, the antisense strand comprises the nucleotides at positions 1-19, 1-20, 1-21, 1-22, 1-23, 2-18, 2-19, 2-20, 2-21, 3-20, or 3-21 from the 5' end of any one of the sequences set forth in SEQ ID NOs: 81-160.

In some embodiments of the present disclosure, the antisense strand comprises the nucleotides at positions 1-19, 1-20, 1-21, 1-22, 1-23, 2-18, 2-19, 2-20, 2-21, 3-20, or 3-21 from the 5' end of any one of the sequences set forth in SEQ ID NOs: 189-230.

In some embodiments of the present disclosure, 1 to 4 nucleotide residues at positions 2, 5-7, 12, and 14-18 from the 5' end of the antisense strand are independently replaced by dR, R, or Z.

In some embodiments of the present disclosure, 1 to 4 nucleotide residues at positions 2, 5-7, and 12 from the 5' end of the antisense strand are independently and optionally replaced by dR, R, or Z.

In some embodiments of the present disclosure, 1 to 4 nucleotides at positions 2, 5-7, 12, and 14-18 from the 5' end of the antisense strand are independently and optionally 2'-deoxynucleotides.

In some embodiments of the present disclosure, 1 to 4 nucleotides at positions 2, 5-7, 12, and 14-18 from the 5' end of the antisense strand are independently and optionally deoxyadenosine, deoxyguanosine, deoxycytidine, or deoxythymidine.

In some embodiments of the present disclosure, 1 to 4 nucleotides at positions 2, 5-7, and 12 from the 5' end of the antisense strand are independently and optionally deoxyadenosine, deoxyguanosine, deoxycytidine, or deoxythymidine.

In some embodiments of the present disclosure, the 5' end or the 3' end of the sense strand or the antisense strand independently and optionally comprises an overhang.

In some embodiments of the present disclosure, the overhang comprises 1 to 5 nucleotides.

In some embodiments of the present disclosure, the overhang comprises 2 nucleotides.

In some embodiments of the present disclosure, the 3' end of the antisense strand optionally comprises an overhang, wherein the overhang comprises 1 to 5 nucleotides.

In some embodiments of the present disclosure, the 3' end of the antisense strand optionally comprises an overhang, wherein the overhang comprises 2 nucleotides.

In some embodiments of the present disclosure, the first nucleoside at the 5' end of the antisense strand may be replaced by a vinylphosphonate-containing nucleoside analog.

In some embodiments of the present disclosure, the vinylphosphonate-containing nucleoside analog is selected from the following structural moieties:

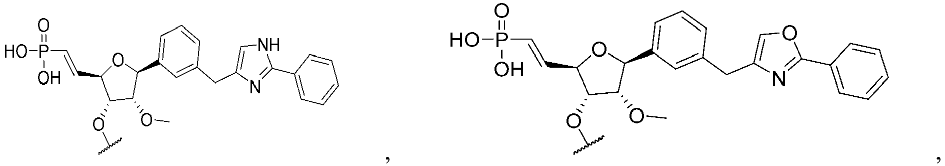

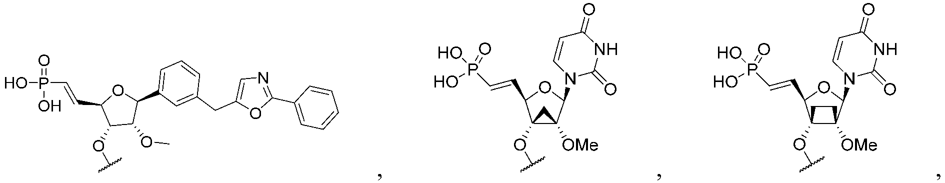

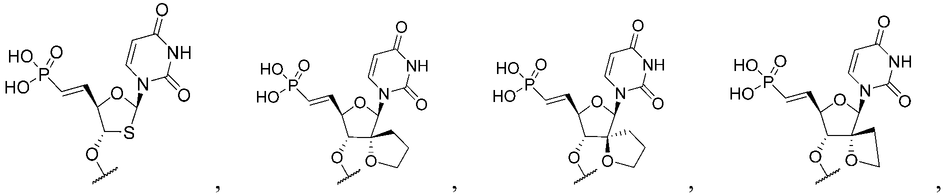

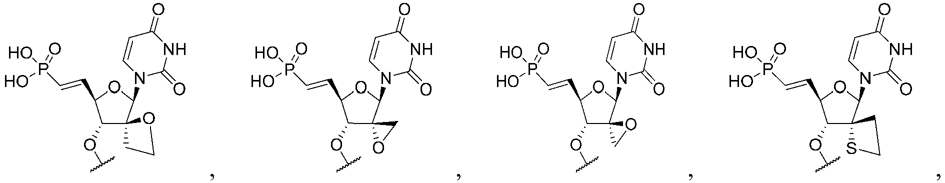

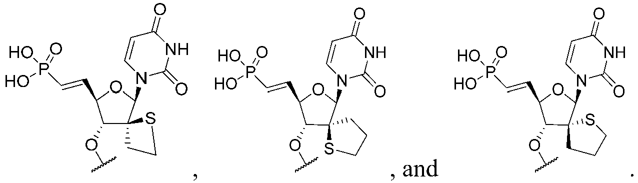

In some embodiments of the present disclosure, the vinylphosphonate-containing nucleoside analog is selected from B19 and B33, wherein B19 is 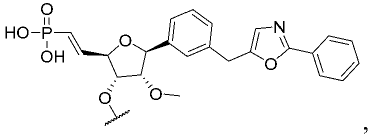 and B33 is

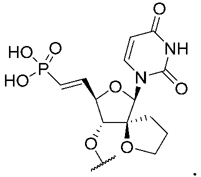

In some embodiments of the present disclosure, the vinylphosphonate-containing nucleoside analog is B33, wherein B33 is

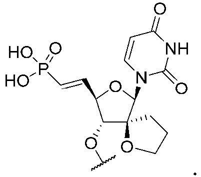

In some embodiments of the present disclosure, the sense strand is selected from any one of the sequences set forth in SEQ ID NOs: 161-188, and the antisense strand is selected from any one of the sequences set forth in SEQ ID NOs: 189-230.

In some embodiments of the present disclosure, the double-stranded siRNA is selected from S1-S81.

The present disclosure provides a double-stranded siRNA or a pharmaceutically acceptable salt thereof, comprising a sense strand and an antisense strand capable of forming a duplex region, wherein the combination of the nucleotide sequence of the sense strand and the nucleotide sequence of the antisense strand is selected from any one of the nucleotide sequence combinations set forth in S83-S129.

In some embodiments of the present disclosure, the double-stranded siRNA is selected from Table 1 and Table 2.

**Table 1: Unmodified double-stranded siRNAs**

| **Double-stranded siRNA compound** | **SEQ ID NO** | **Sense strand (5'-3')** | **SEQ ID NO** | **Antisense strand (5'-3')** |
|---|---|---|---|---|
| S1 | 1 | AGCUAGCCAAGCAGCAA AUCA | 81 | UGAUUUGCUGCUUGGCUA GCUCC |
| S2 | 2 | CCAGUCGUCCCAGAAUA ACUA | 82 | UAGUUAUUCUGGGACGAC UGGUC |
| S3 | 3 | CGAGACCAUUACGUAGA CUUA | 83 | UAAGUCUACGUAAUGGUC UCGCC |
| S4 | 4 | AAUGUGGUCAAGACGGA UGUA | 84 | UACAUCCGUCUUGACCAC AUUGC |
| S5 | 5 | GCUAGCCAAGCAGCAAA UCCA | 85 | UGGAUUUGCUGCUUGGCU AGCUC |
| S6 | 6 | CUCCUCAAAGCCAACAA UCCA | 86 | UGGAUUGUUGGCUUUGAG GAGGC |
| S7 | 7 | GUCUUCAGCCUCCUCAA AGCA | 87 | UGCUUUGAGGAGGCUGAA GACGG |
| S8 | 8 | UGGAUCAUAAUGGCAAU GUGA | 88 | UCUCAUUGCCAUUAUGAU CCAGG |
| S9 | 9 | UCUACCUGGAUCAUAAU GGCA | 89 | UGCCAUUAUGAUCCAGGU AGAGG |
| S10 | 10 | GACGGAUGUGCCAGAUA UGGA | 90 | UCCAUAUCUGGCACAUCC GUCUU |
| S11 | 11 | UCAAGACGGAUGUGCCA GAUA | 91 | UAUCUGGCACAUCCGUCU UGACC |
| S12 | 12 | AUCAUAAUGGCAAUGUG GUCA | 92 | UGACCACAUUGCCAUUAU GAUCC |
| S13 | 13 | UUAUGUUGCAGGCGAGA CCAU | 93 | AUGGUCUCGCCUGCAACA UAAGG |
| S14 | 14 | CCCCUCUCUCUCCUCUAC CUA | 94 | UAGGUAGAGGAGAGAGAG GGGCC |
| S15 | 15 | UCUUCAGCCUCCUCAAA GCCA | 95 | UGGCUUUGΛGGΛGGCUGΛ AGACG |
| S16 | 16 | CUCUACCUGGAUCAUAA UGGA | 96 | UCCAUUAUGAUCCAGGUA GAGGA |
| S17 | 17 | GACCCCCUUAUGUUGCA GGCA | 97 | UGCCUGCAACAUAAGGGG GUCGC |
| S18 | 18 | CCUUAUGUUGCAGGCGA GACA | 98 | UGUCUCGCCUGCAACAUA AGGGG |
| S19 | 19 | CUCUGGUGCUGGAGCUA GCCA | 99 | UGGCUAGCUCCAGCACCA GAGCU |
| S20 | 20 | GGCUCUUGGACACAGCA GGAA | 100 | UUCCUGCUGUGUCCAAGA GCCGC |
| S21 | 21 | GGGUACCAGCUGAAUUA CUGA | 101 | UCAGUAAUUCAGCUGGUA CCCCU |
| S22 | 22 | GCUUAAGAUCCGAGCCA AUGA | 102 | UCAUUGGCUCGGAUCUUA AGCUC |
| S23 | 23 | GUGCUGGAGCUAGCCAA GCAA | 103 | UUGCUUGGCUAGCUCCAG CACCA |
| S24 | 24 | CUGGAUCAUAAUGGCAA UGUA | 104 | UACAUUGCCAUUAUGAUC CAGGU |
| S25 | 25 | CAGUCGUCCCAGAAUAA CUCA | 105 | UGAGUUAUUCUGGGACGA CUGGU |
| S26 | 26 | AAUGGCAAUGUGGUCAA GACA | 106 | UGUCUUGACCACAUUGCC AUUAU |
| S27 | 27 | CACCCCAAGCAGAACGA GCUA | 107 | UAGCUCGUUCUGCUUGGG GUGCC |
| S28 | 28 | UGACCAGUCGUCCCAGA AUAA | 108 | UUAUUCUGGGACGACUGG UCAGG |
| S29 | 29 | UCCUCAAAGCCAACAAU CCUU | 109 | AAGGAUUGUUGGCUUUGA GGAGG |
| S30 | 30 | GCCCUCCGGAGACUACA GCCA | 110 | UGGCUGUAGUCUCCGGAG GGCUC |
| S31 | 31 | UCCCACCACCUGUACCAU GCA | 111 | UGCAUGGUACAGGUGGUG GGACC |
| S32 | 32 | AGGGGUACCAGCUGAAU UACU | 112 | AGUAAUUCAGCUGGUACC CCUCG |
| S33 | 33 | GAGCCCUCCGGAGACUA CAGA | 113 | UCUGUAGUCUCCGGAGGG CUCUG |
| S34 | 34 | CGGUCCCACCACCUGUAC CAU | 114 | AUGGUACAGGUGGUGGGA CCGAG |
| S35 | 35 | GUGGUCAAGACGGAUGU GCCA | 115 | UGGCACAUCCGUCUUGAC CACAU |
| S36 | 36 | UCAAAGCCAACAAUCCU UGGA | 116 | UCCAAGGAUUGUUGGCUU UGAGG |
| S37 | 37 | CCUCUACCUGGAUCAUA AUGA | 117 | UCAUUAUGAUCCAGGUAG AGGAG |
| S38 | 38 | CCAGAGCCCUCCGGAGA CUAA | 118 | UUAGUCUCCGGAGGGCUC UGGUC |
| S39 | 39 | AGCUGAAUUACUGCAGU GGGA | 119 | UCCCACUGCAGUAAUUCA GCUGG |
| S40 | 40 | GGCAUUGCUGCCUCUUU CCAU | 120 | AUGGAAAGAGGCAGCAAU GCCUG |
| S41 | 41 | GGGGUACCAGCUGAAUU ACUA | 121 | UAGUAAUUCAGCUGGUAC CCCUC |
| S42 | 42 | AGCCUCCUCAAAGCCAA CAAU | 122 | AUUGUUGGCUUUGAGGAG GCUGA |
| S43 | 43 | AUGUGGUCAAGACGGAU GUGA | 123 | UCACAUCCGUCUUGACCA CAUUG |
| S44 | 44 | UGCUGGAGCUAGCCAAG CAGA | 124 | UCUGCUUGGCUAGCUCCA GCACC |
| S45 | 45 | GACCAGUCGUCCCAGAA UAAA | 125 | UUUAUUCUGGGACGACUG GUCAG |
| S46 | 46 | AGGACCCCCACCUGUGA GCCU | 126 | AGGCUCACAGGUGGGGU CCUCC |
| S47 | 47 | UGCGΛCCCCCUUΛUGUU GCAA | 127 | UUGCAACAUAAGGGGGUC GCAGG |
| S48 | 48 | AUUGCUGCCUCUUUCCA UUCU | 128 | AGAAUGGAAAGAGGCAGC AAUGC |
| S49 | 49 | CACCUGUGAGCCUGCGA CCCA | 129 | UGGGUCGCAGGCUCACAG GUGGG |
| S50 | 50 | CUCCUCUACCUGGAUCA UAAU | 130 | AUUAUGAUCCAGGUAGAG GAGAG |
| S51 | 51 | CGAGCUCUGGUGCUGGA GCUA | 131 | UAGCUCCAGCACCAGAGC UCGUU |
| S52 | 52 | GAGGGUACCAGCUGAA UUAA | 132 | UUAAUUCAGCUGGUACCC CUCGG |
| S53 | 53 | AAGCAGCAAAUCCUGGA UGGA | 133 | UCCAUCCAGGAUUUGCUG CUUGG |
| S54 | 54 | CUGGCAGCCAGGCAUU GCUA | 134 | UAGCAAUGCCUGGGCUGC CAGCC |
| S55 | 55 | GACCAUUACGUAGACUU CCAA | 135 | UUGGAAGUCUACGUAAUG GUCUC |
| S56 | 56 | CAAAGCCAACAAUCCUU GGCA | 136 | UGCCAAGGAUUGUUGGCU UUGAG |
| S57 | 57 | CGAGGGGUACCAGCUGA AUUA | 137 | UAAUUCAGCUGGUACCCC UCGGG |
| S58 | 58 | CUUAUGUUGCAGGCGAG ACCA | 138 | UGGUCUCGCCUGCAACAU AAGGG |
| S59 | 59 | UCGGUCCCΛCCΛCCUGU ACCA | 139 | UGGUACAGGUGGUGGGAC CGAGG |
| S60 | 60 | GUACCAGCUGAAUUACU GCAA | 140 | UUGCAGUAAUUCAGCUGG UACCC |
| S61 | 61 | AGGCAGCGCUGACCAGA GCCA | 141 | UGGCUCUGGUCΛGCGCUG CCUGG |
| S62 | 62 | GAGACCAUUACGUAGAC UUCA | 142 | UGAAGUCUACGUAAUGGU CUCGC |
| S63 | 63 | UCCGAGCCAAUGAGCCU GGAA | 143 | UUCCAGGCUCAUUGGCUC GGAUC |
| S64 | 64 | GGUGCUGGAGCUAGCCA AGCA | 144 | UGCUUGGCUAGCUCCAGC ACCAG |
| S65 | 65 | UAAUGGCAAUGUGGUCA AGAA | 145 | UUCUUGACCACAUUGCCA UUAUG |
| S66 | 66 | GCUCUGGUGCUGGAGCU AGCA | 146 | UGCUAGCUCCAGCACCAG AGCUC |
| S67 | 67 | GCUGAAUUACUGCAGUG GGCA | 147 | UGCCCACUGCAGUAAUUC AGCUG |
| S68 | 68 | CUAGCCAAGCAGCAAAU CCUA | 148 | UAGGAUUUGCUGCUUGGC UAGCU |
| S69 | 69 | CGUCUUCAGCCUCCUCA AAGA | 149 | UCUUUGAGGAGGCUGAAG ACGGC |
| S70 | 70 | ACCAGUCGUCCCAGAAU AACU | 150 | AGUUAUUCUGGGACGACU GGUCA |
| S71 | 71 | ACCAUUACGUAGACUUC CAGA | 151 | UCUGGAAGUCUACGUAAU GGUCU |
| S72 | 72 | AGACGGAUGUGCCAGAU AUGA | 152 | UCAUAUCUGGCACAUCCG UCUUG |
| S74 | 73 | GAGGGUACCAGCUGAA UUAC | 153 | GUAAUUCAGCUGGUACCC CUCGG |
| S75 | 74 | CAGAGCCCUCCGGAGAC UACA | 154 | UGUΛGUCUCCGGΛGGGCU CUGGU |
| S76 | 75 | AGACCAUUACGUAGACU UCCA | 155 | UGGAAGUCUACGUAAUGG UCUCG |
| S77 | 76 | ACCAUUACGUAGACUUC CAGG | 156 | CCUGGAAGUCUACGUAAU GGUCU |
| S78 | 77 | CCCGAGGGUACCAGCU GAAU | 157 | AUUCAGCUGGUACCCCUC GGGCU |
| S79 | 78 | GCACCCCAAGCAGAACG AGCU | 158 | AGCUCGUUCUGCUUGGGG UGCCA |
| S80 | 79 | ACUGGCACCCCAAGCAG AACG | 159 | CGUUCUGCUUGGGUGCC AGUUU |
| S81 | 80 | ACCCCAAGCAGAACGAG CUCU | 160 | AGAGCUCGUUCUGCUUGG GGUGC |

**Table 2: Modified double-stranded siRNAs**

| **Double-stranded siRNA compound** | **SEQ ID NO** | **Sense strand (5'-3')** | **SEQ ID NO** | **Antisense strand (5'-3')** |
|---|---|---|---|---|
| S83 | 161 | cs,cs,a,g,u,c,g,u,Cf,Cf,Cf,a,g,a, a,u,a,a,c,u,a | 189 | us,dAs,g,u,dT,a,dT,u,c,u,g,dG,g, Af,c,g,a,c,u,g,gs,us,c |
| S84 | 162 | as,as,u,g,u,g,g,u,Cf,Af,Af,g,a,c ,g,g,a,u,g,u,a | 190 | us,dAs,c,a,dT,c,dC,g,u,c,u,dT,g, Af,c,c,a,c,a,u,us,gs,c |
| S85 | 163 | as,gs,g,g,g,u,a,c,Cf,Af,Gf,c,u,g ,a,a,u,u,a,c,u | 191 | as,dGs,u,a,dA,u,dT,c,a,g,c,dT,g, Gf,u,a,c,c,c,c,us,cs,g |
| S86 | 164 | gs,us,g,g,u,c,a,a,Gf,Af,Cf,g,g,a ,u,g,u,g,c,c,a | 192 | us,dGs,g,c,dA,c,dA,u,c,c,g,dT,c, Uf,u,g,a,c,c,a,cs,as,u |
| S87 | 165 | cs,cs,u,c,u,a,c,c,Uf,Gf,Gf,a,u,c, a,u,a,a,u,g,a | 193 | us,dCs,a,u,dT,a,dT,g,a,u,c,dC,a, Gf,g,u,a,g,a,g,gs,as,g |
| S88 | 166 | gs,gs,g,g,u,a,c,c,Af,Gf,Cf,u,g,a ,a,u,u,a,c,u,a | 194 | us,dAs.g,u,dA,a,dT,u,c,a,g,dC,u, Gf,g,u,a,c,c,c,cs,us,c |
| S89 | 167 | as,gs,c,c,u,c,c,u,Cf,Af,Af,a,g,c, c,a,a,c,a,a,u | 195 | as,dTs,u,g,dT,u,dG,g,c,u,u,dT,g, Af,g,g,a,g,g,c,us,gs,a |
| S90 | 168 | gs,as,c,c,a,g,u,c,Gf,Uf,Cf,c,c,a, g,a,a,u,a,a,a | 196 | us,dTs,u,a,dT,u,dC,u,g,g,g,dA,c, Gf,a,c,u,g,g,u,cs,as,g |
| S91 | 169 | gs,as,c,c,a,u,u,a,Cf,Gf,Uf,a,g,a, c,u,u,c,c,a,a | 197 | us,dTs,g,g,dA,a,dG,u,c,u,a,dC,g, Uf,a,a,u,g,g,u,cs,us,c |
| S92 | 170 | cs,gs,a,g,g,g,g,u,Af,Cf,Cf,a,g,c ,u,g,a,a,u,u,a | 198 | us,dAs,a,u,dT,c,dA,g,c,u,g,dG,u, Af,c,c,c,c,u,c,gs,gs,g |
| S93 | 171 | as,cs,c,a,g,u,c,g,Uf,Cf,Cf,c,a,g, a,a,u,a,a,c,u | 199 | as,dGs,u,u,dA,u,dT,c,u,g,g,dG,a, Cf,g,a,c,u,g,g,us,cs,a |
| S94 | 172 | cs,us,c,c,u,c,u,a,Cf,Cf,Uf,g,g,a, u,c,a,u,a,a,u | 200 | as,dTs,u,a,dT,g,dA,u,c,c,a,dG,g, Uf,a,g,a,g,g,a,gs,as,g |
| S95 | 161 | cs,cs,a,g,u,c,g,u,Cf,Cf,Cf,a,g,a, a,u,a,a,c,u,a | 201 | us,dAs,g,u,dT,a,Z,u,c,u,g,dG,g, Af,c,g,a,c,u,g,gs,us,c |
| S96 | 161 | cs,cs,a,g,u,c,g,u,Cf,Cf,Cf,a,g,a, a,u,a,a,c,u,a | 202 | us,dAs,g,u,Z,a,dT,u,c,u,g,dG,g, Af,c,g,a,c,u,g,gs,us,c |
| S97 | 161 | cs,cs,a,g,u,c,g,u,Cf,Cf,Cf,a,g,a, a,u,a,a,c,u,a | 203 | us,as,g,u,dT,a,Z,u,c,u,g,dG,g,Af, c,g,a,c,u,g,gs,us,c |
| S98 | 161 | cs,cs,a,g,u,c,g,u,Cf,Cf,Cf,a,g,a, a,u,a,a,c,u,a | 204 | us,dAs,g,u,dT,a,Z,u,c,u,g,Gf,g,A f,c,g,a,c,u,g,gs,us,c |
| S99 | 161 | cs,cs,a,g,u,c,g,u,Cf,Cf,Cf,a,g,a, a,u,a,a,c,u,a | 205 | B19s,dAs,g,u,Z,a,dT,u,c,u,g,dG, g,Af,c,g,R,c,u,g,gs,us,c |
| S100 | 161 | cs,cs,a,g,u,c,g,u,Cf,Cf,Cf,a,g,a, a,u,a,a,c,u,a | 206 | B19s,dAs,g,u,Z,a,dT,u,c,u,g,dG, g,Af,c,g,R,c,u,g,gs,cs,c |
| S101 | 173 | cs,cs,a,g,u,c,g,u,Cf,Cf,Cf,a,g,a, a,u,a,R,c,u,a | 205 | B19s,dAs,g,u,Z,a,dT,u,c,u,g,dG, g,Af,c,g,R,c,u,g,gs,us,c |
| S102 | 174 | cs,cs,R,g,u,c,g,u,Cf,Cf,Cf,a,g,a ,a,u,a,R,c,u,a | 205 | B19s,dAs,g,u,Z,a,dT,u,c,u,g,dG, g,Af,c,g,R,c,u,g,gs,us,c |
| S103 | 161 | cs,cs,a,g,u,c,g,u,Cf,Cf,Cf,a,g,a, a,u,a,a,c,u,a | 230 | us,dAs,g,u,Z,a,dT,u,c,u,g,dG,g, Af,c,g,a,c,R,g,gs,us,c |
| S104 | 161 | cs,cs,a,g,u,c,g,u,Cf,Cf,Cf,a,g,a, a,u,a,a,c,u,a | 207 | us,dAs,g,u,Z,a,dT,u,c,u,g,dG,g, Af,c,g,a,c,R,g,gs,cs,c |
| S105 | 161 | cs,cs,a,g,u,c,g,u,Cf,Cf,Cf,a,g,a, a,u,a,a,c,u,a | 208 | us,dAs,g,u,dR,a,dT,u,c,u,g,dG,g, Af,c,g,a,c,R,g,gs,us,c |
| S106 | 161 | cs,cs,a,g,u,c,g,u,Cf,Cf,Cf,a,g,a, a,u,a,a,c,u,a | 209 | us,dAs,g,u,dR,a,dR,u,c,u,g,dG,g, Af,c,g,a,c,R,g,gs,us,c |
| S107 | 161 | cs,cs,a,g,u,c,g,u,Cf,Cf,Cf,a,g,a, a,u,a,a,c,u,a | 210 | us,dAs,g,u,dT,a,dR,u,c,u,g,dG,g, Af,c,g,a,c,R,g,gs,us,c |
| S109 | 175 | gs,as,g,g,g,g,u,a,Cf,Cf,Af,g,c,u ,g,a,a,u,u,a,a | 211 | us,dTs,a,a,dT,u,dC,a,g,c,u,dG,g, Uf,a,c,c,c,c,u,cs,gs,g |
| S110 | 176 | as,gs,g,g,g,u,a,Cf,Cf,Af,g,c,u,g ,a,a,u,u,a,c,a | 212 | us,dGs,u,a,dA,u,dT,c,a,g,c,dT,g, Gf,u,a,c,c,c,c,us,cs,g |
| S111 | 177 | cs,as,g,a,g,c,c,c,Uf,Cf,Cf,g,g,a, g,a,c,u,a,c,a | 213 | us,dGs,u,a,dG,u,dC,u,c,c,g,dG,a, Gf,g,g,c,u,c,u,gs,gs,u |
| S112 | 178 | as,gs,a,c,c,a,u,u,Af,Cf,Gf,u,a,g ,a,c,u,u,c,c,a | 214 | us,dGs,g,a,dA,g,dT,c,u,a,c,dG,u, Af,a,u,g,g,u,c,us,cs,g |
| S113 | 179 | as,cs,c,a,u,u,a,c,Gf,Uf,Af,g,a,c, u,u,c,c,a,g,a | 215 | us,dCs,u,g,dG,a,dA,g,u,c,u,dA,c, Gf,u,a,a,u,g,g,us,cs,u |
| S114 | 180 | cs,cs,c,g,a,g,g,g,Gf,Uf,Af,c,c,a ,g,c,u,g,a,a,a | 216 | us,dTs,u,c,dA,g,dC,u,g,g,u,dA,c, Cf,c,c,u,c,g,g,gs,cs,u |
| S115 | 181 | gs,cs,a,c,c,c,c,a,Af,Gf,Cf,a,g,a, a,c,g,a,g,c,a | 217 | us,dGs,c,u,dC,g,dT,u,c,u,g,dC,u, Uf,g,g,g,g,u,g,cs,cs,u |
| S116 | 182 | as,cs,u,g,g,c,a,c,Cf,Cf,Cf,a,a,g, c,a,g,a,a,c,a | 218 | us,dGs,u,u,dC,u,dG,c,u,u,g,dG,g ,Gf,u,g,c,c,a,g,us,us,u |
| S117 | 183 | as,cs,c,c,c,a,a,g,Cf,Af,Gf,a,a,c, g,a,g,c,u,c,a | 219 | us,dGs,a,g,dC,u,dC,g,u,u,c,dT,g, Cf,u,u,g,g,g,g,us,gs,u |
| S118 | 184 | as,gs,a,c,c,a,u,u,Af,Cf,Gf,u,a,g ,a,c,Uf,u,c,c,a | 220 | U,us,dGs,gs,a,dA,g,dT,c,u,a,c,d G,u,Af,a,u,g,g,u,cs,us,cs,g |
| S119 | 184 | as,gs,a,c,c,a,u,u,Af,Cf,Gf,u,a,g ,a,c,Uf,u,c,c,a | 221 | B33s,Gfs,gs,a,dA,g,dT,c,u,a,c,Gf ,u,Af,a,Uf,g,Gf,u,Cfs,us,Cfs,g |
| S120 | 184 | as,gs,a,c,c,a,u,u,Af,Cf,Gf,u,a,g ,a,c,Uf,u,c,c,a | 222 | us,dGs,gs,a,dA,g,dR,c,u,a,c,dG, u,Af,a,u,g,g,u,cs,us,cs,g |
| S121 | 184 | as,gs,a,c,c,a,u,u,Af,Cf,Gf,u,a,g ,a,c,Uf,u,c,c,a | 223 | B33s,dGs,gs,a,dA,g,dR,c,u,a,c,d G,u,Af,a,u,g,g,u,cs,us,cs,g |
| S122 | 184 | as,gs,a,c,c,a,u,u,Af,Cf,Gf,u,a,g ,a,c,Uf,u,c,c,a | 224 | B33s,Gfs,gs,a,dA,g,Z,c,u,a,c,Gf, u,Af,a,Uf,g,Gf,u,Cfs,us,Cfs,g |
| S123 | 184 | as,gs,a,c,c,a,u,u,Af,Cf,Gf,u,a,g ,a,c,Uf,u,c,c,a | 225 | B33s,Gfs,g,a,Af,g,Uf,c,u,a,c,g,u, Af,a,Uf,g,Gf,u,c,us,cs,g |
| S124 | 185 | as,gs,a,c,c,Af,Uf,u,Af,Cf,Gf,u, a,g,a,c,Uf,u,c,c,a | 220 | U,us,dGs,gs,a,dA,g,dT,c,u,a,c,d G,u,Af,a,u,g,g,u,cs,us,cs,g |
| S125 | 186 | cs,cs,Af,Uf,u,Af,Cf,Gf,u,a,g,a, c,Uf,u,c,c,a | 226 | us,dGs,gs,a,dA,g,dT,c,u,a,c,dG,u ,Af,a,u,g,g,u,cs,us,cs,g |
| S126 | 187 | cs,cs,Af,Uf,u,Af,Cf,Gf,u,a,g,a, c,u,u,c,c,a | 227 | B33s,dGs,gs,a,dA,g,dT,c,u,a,c,d G,u,Af,a,u,g,g,u,cs,us,cs,g |
| S127 | 188 | as,gs,a,c,c,Af,Uf,u,Af,Cf,Gf,u, a,g,a,c,u,u,c,c,a | 228 | B33s,dGs,gs,a,dA,g,dT,c,u,a,c,g, u,Af,a,u,g,g,u,cs,us,cs,g |
| S128 | 188 | as,gs,a,c,c,Af,Uf,u,Af,Cf,Gf,u, a,g,a,c,u,u,c,c,a | 229 | B33s,dGs,gs,a,dA,g,dR,c,u,a,c,g, u,Af,a,u,g,g,u,cs,us,cs,g |
| S129 | 188 | as,gs,a,c,c,Af,Uf,u,Af,Cf,Gf,u, a,g,a,c,u,u,c,c,a | 225 | B33s,Gfs,g,a,Af,g,Uf,c,u,a,c,g,u, Af,a,Uf,g,Gf,u,c,us,cs,g |

The present disclosure provides a conjugate or a pharmaceutically acceptable salt thereof, comprising any one of the double-stranded siRNAs described above and a pharmaceutically acceptable conjugate moiety.

In some embodiments of the present disclosure, the conjugate or a pharmaceutically acceptable salt thereof may comprise 1, 2, 3, 4, or 5 pharmaceutically acceptable conjugate moieties.

In some embodiments of the present disclosure, the conjugate or a pharmaceutically acceptable salt thereof comprises one pharmaceutically acceptable conjugate moiety.

In some embodiments of the present disclosure, the pharmaceutically acceptable conjugate moiety is selected from an antibody, a polypeptide, and a ligand.

In some embodiments of the present disclosure, the pharmaceutically acceptable conjugate moiety may be independently attached to any nucleotide of the sense strand and/or the antisense strand of the double-stranded siRNA.

In some embodiments of the present disclosure, the pharmaceutically acceptable conjugate moiety may be attached to the 3' end and/or the 5' end of the sense strand and/or the antisense strand of the double-stranded siRNA.

In some embodiments of the present disclosure, the pharmaceutically acceptable conjugate moiety independently comprises 1, 2, 3, 4, or 5 GalNAc moieties, wherein the GalNAc moieties are capable of binding to ASGPR.

In some embodiments of the present disclosure, the conjugate moiety is selected from the following structures: 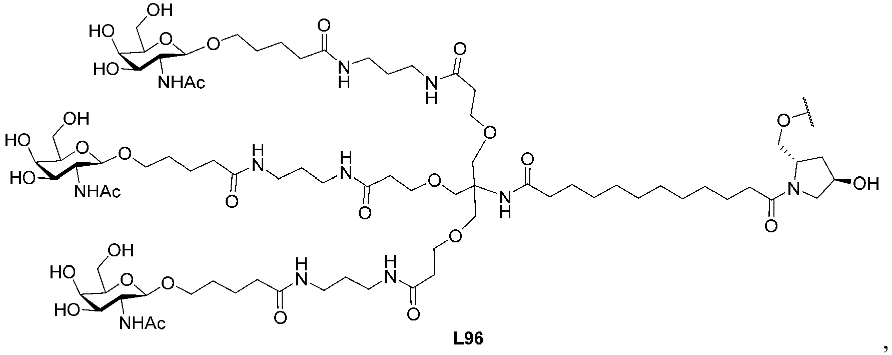 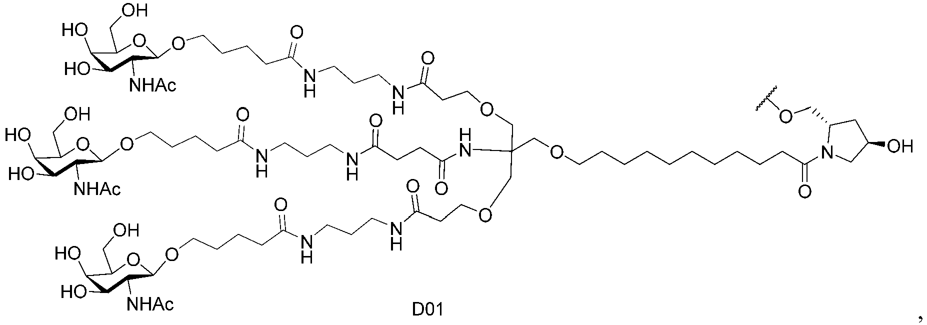 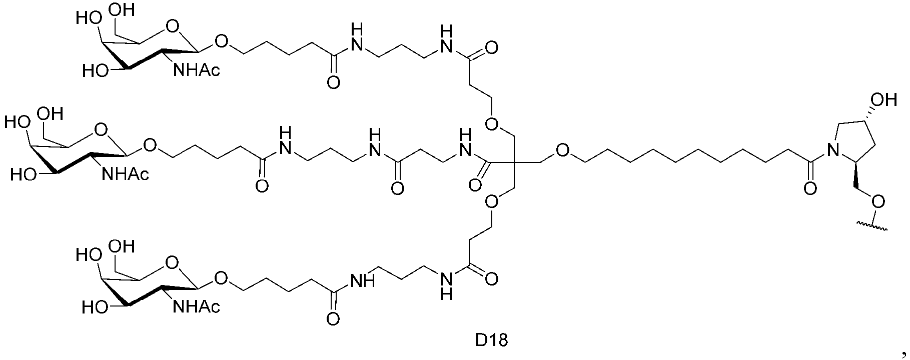 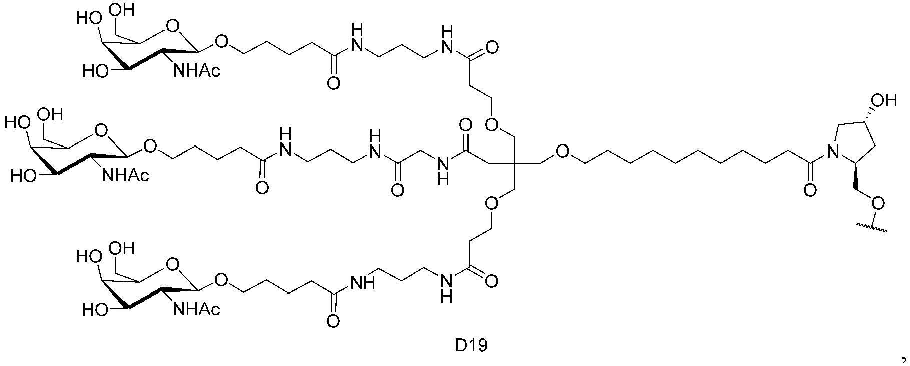 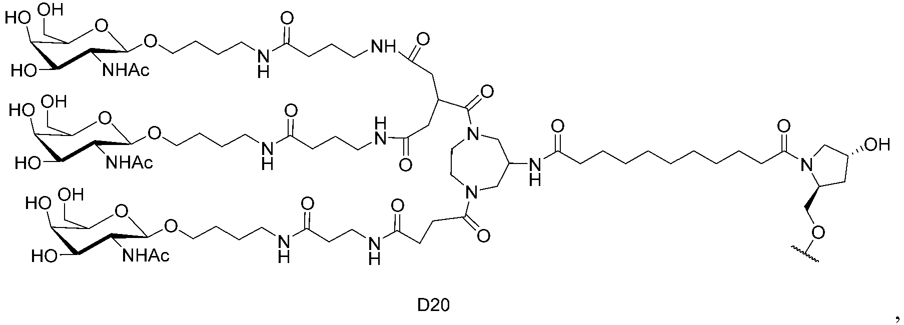 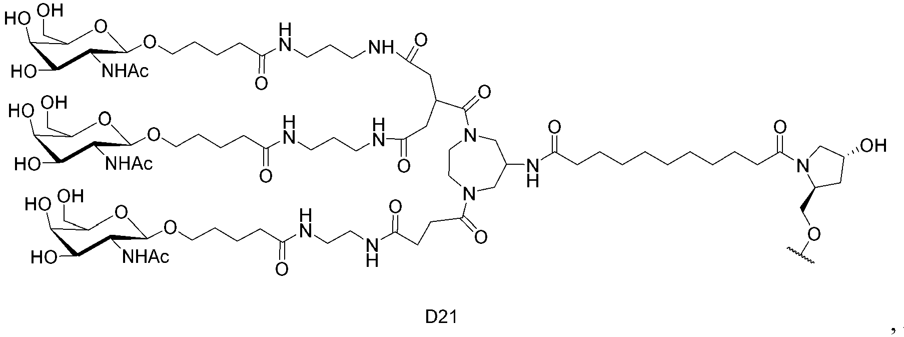 and

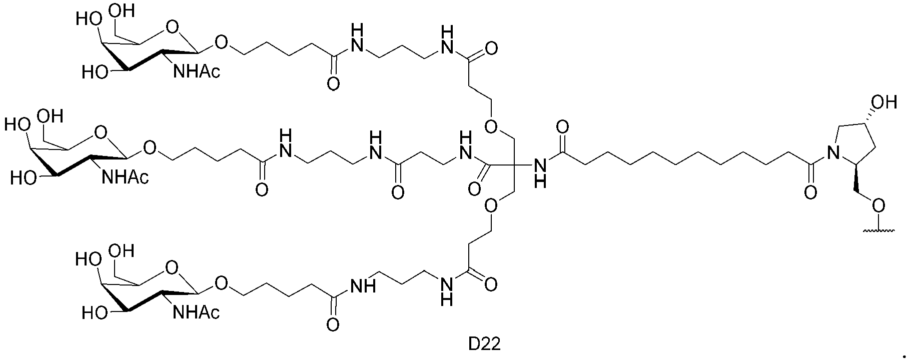

In the present disclosure, the mode of linkage between the double-stranded siRNA and the conjugate moiety in the conjugate is as follows: 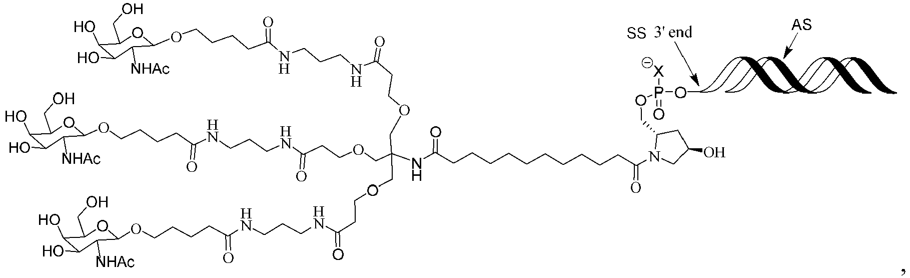 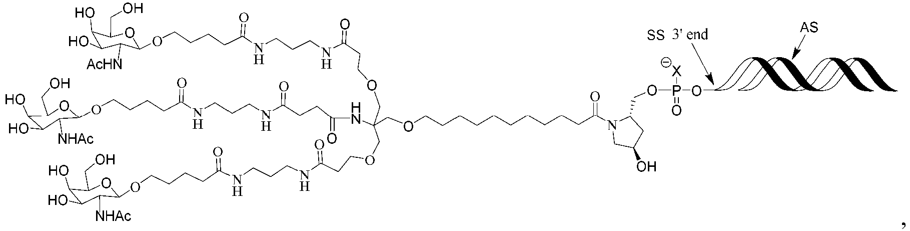 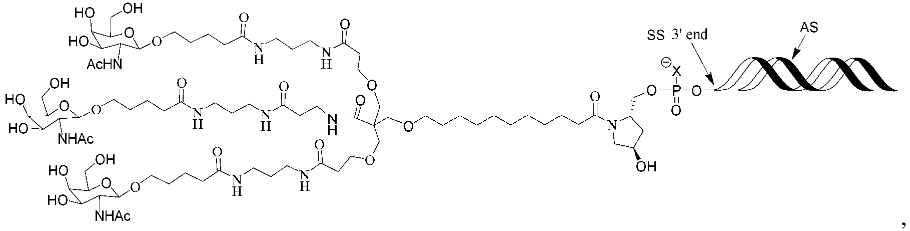 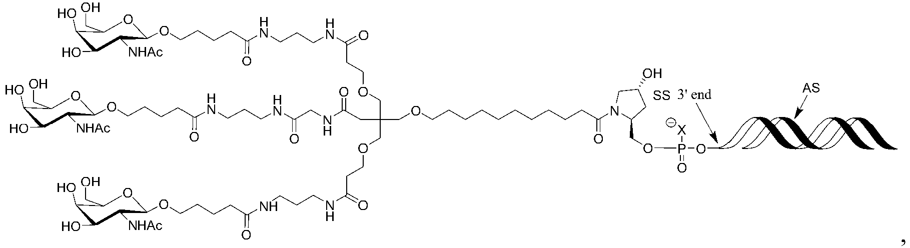 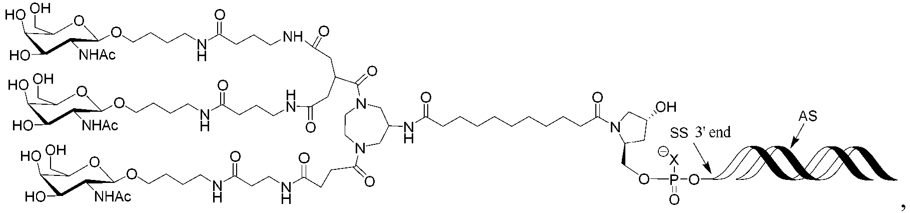 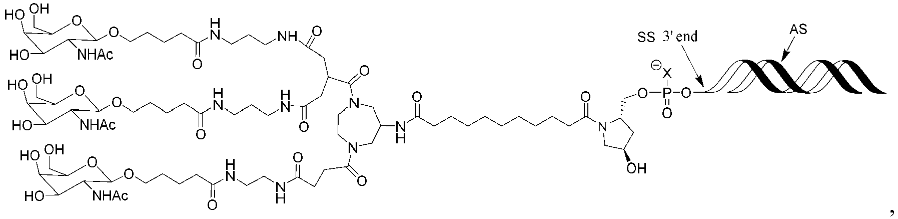 and 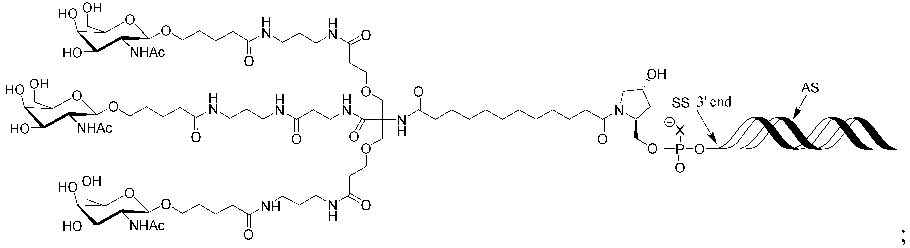 wherein SS represents the sense strand and AS represents the antisense strand; X is selected from O and S.

In some embodiments of the present disclosure, the conjugate is selected from C1-C47.

In some embodiments of the present disclosure, the conjugate is selected from Table 3.

**Table 3: Conjugates**

| **Conjugate** | **Sense strand sequence SEQ ID NO** | **Conjugate moiety** (conjugated to the 3' end of the sense strand via a phosphate linkage or a phosphorothioate linkage) | **Antisense strand sequence SEQ ID NO** |
|---|---|---|---|
| C1 | 161 | D01 | 189 |
| C2 | 162 | D01 | 190 |
| C3 | 163 | D01 | 191 |
| C4 | 164 | D01 | 192 |
| C5 | 165 | D01 | 193 |
| C6 | 166 | D01 | 194 |
| C7 | 167 | D01 | 195 |
| C8 | 168 | D01 | 196 |
| C9 | 169 | D01 | 197 |
| C10 | 170 | D01 | 198 |
| C11 | 171 | D01 | 199 |
| C12 | 172 | D01 | 200 |
| C13 | 161 | D01 | 201 |
| C14 | 161 | D01 | 202 |
| C15 | 161 | D01 | 203 |
| C16 | 161 | D01 | 204 |
| C17 | 161 | D01 | 205 |
| C18 | 161 | D01 | 206 |
| C19 | 173 | D01 | 205 |
| C20 | 174 | D01 | 205 |
| C21 | 161 | D01 | 230 |
| C22 | 161 | D01 | 207 |
| C23 | 161 | D01 | 208 |
| C24 | 161 | D01 | 209 |
| C25 | 161 | D01 | 210 |
| C27 | 175 | D01 | 211 |
| C28 | 176 | D01 | 212 |
| C29 | 177 | D01 | 213 |
| C30 | 178 | D01 | 214 |
| C31 | 179 | D01 | 215 |
| C32 | 180 | D01 | 216 |
| C33 | 181 | D01 | 217 |
| C34 | 182 | D01 | 218 |
| C35 | 183 | D01 | 219 |
| C36 | 184 | D01 | 220 |
| C37 | 184 | D01 | 221 |
| C38 | 184 | D01 | 222 |
| C39 | 184 | D01 | 223 |
| C40 | 184 | D01 | 224 |
| C41 | 184 | D01 | 225 |
| C42 | 185 | D01 | 220 |
| C43 | 186 | D01 | 226 |
| C44 | 187 | D01 | 227 |
| C45 | 188 | D01 | 228 |
| C46 | 188 | D01 | 229 |
| C47 | 188 | D01 | 225 |

In some embodiments of the present disclosure, the conjugate is represented by formula (I): 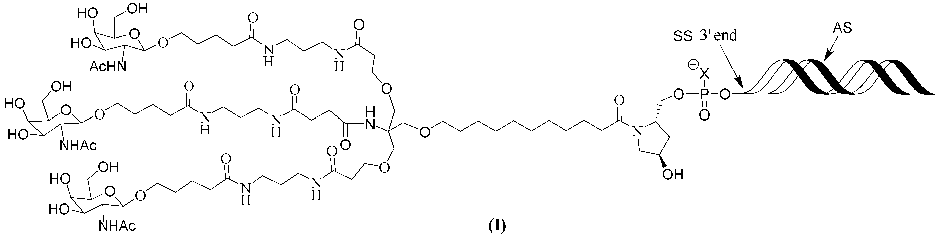 wherein X is O or S; SS represents the sense strand and AS represents the antisense strand; the sense strand and the antisense strand are selected from any one of the following combinations (1) to (9):
(1) the sense strand is set forth in SEQ ID NO: 161, and the antisense strand is set forth in SEQ ID NO: 189;
(2) the sense strand is set forth in SEQ ID NO: 161, and the antisense strand is set forth in SEQ ID NO: 210;
(3) the sense strand is set forth in SEQ ID NO: 186, and the antisense strand is set forth in SEQ ID NO: 226;
(4) the sense strand is set forth in SEQ ID NO: 188, and the antisense strand is set forth in SEQ ID NO: 225;
(5) the sense strand is set forth in SEQ ID NO: 164, and the antisense strand is set forth in SEQ ID NO: 192;
(6) the sense strand is set forth in SEQ ID NO: 171, and the antisense strand is set forth in SEQ ID NO: 199;
(7) the sense strand is set forth in SEQ ID NO: 161, and the antisense strand is set forth in SEQ ID NO: 202;
(8) the sense strand is set forth in SEQ ID NO: 185, and the antisense strand is set forth in SEQ ID NO: 220;
(9) the sense strand is set forth in SEQ ID NO: 188, and the antisense strand is set forth in SEQ ID NO: 228.

In some embodiments of the present disclosure, the conjugate is represented by formula (I): 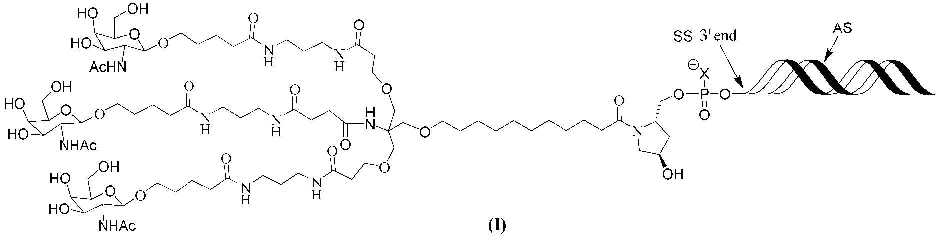 wherein X is O; SS represents the sense strand and AS represents the antisense strand; the sense strand and the antisense strand are selected from any one of the following combinations (1) to (4):
(1) the sense strand is set forth in SEQ ID NO: 161, and the antisense strand is set forth in SEQ ID NO: 189;
(2) the sense strand is set forth in SEQ ID NO: 161, and the antisense strand is set forth in SEQ ID NO: 210;
(3) the sense strand is set forth in SEQ ID NO: 186, and the antisense strand is set forth in SEQ ID NO: 226;
(4) the sense strand is set forth in SEQ ID NO: 188, and the antisense strand is set forth in SEQ ID NO: 225.

The present disclosure further provides the use of the double-stranded siRNA described above, a conjugate thereof, a salt thereof, or a salt of the conjugate thereof, in the manufacture of a medicament for treating a disease associated with INHBE gene expression.

In some embodiments of the present disclosure, in the use described above, the disease associated with INHBE gene expression is a metabolic disease caused by abnormal body fat distribution.

In some embodiments of the present disclosure, in the use described above, the metabolic disease caused by abnormal body fat distribution includes, but is not limited to, type 2 diabetes, obesity, abdominal obesity, coronary heart disease, non-alcoholic fatty liver disease, non-alcoholic steatohepatitis, hyperlipidemia, dyslipidemia, and atherosclerosis.

The present disclosure further provides the following test methods.

### Test method 1: Free uptake assay in primary human hepatocytes (PHHs)

### 1. Experimental principle:

The degree of reduction in INHBE mRNA levels induced by the test compounds was evaluated by incubating the test compounds with PHHs.

### 2. Experimental materials:

Primary human hepatocytes (PHHs); RNeasy^{®} 96 Kit; FastKing RT Kit (With gDNase); TaqMan Gene Expression Assay.

### 3. Experimental procedure:

The conjugates of the present disclosure were diluted with PBS to 10-fold the test concentrations. 10 µL of each siRNA solution was transferred to a 96-well plate. PHHs were thawed and seeded into the 96-well plate at a final density of 5.4 × 10⁵ cells/well. The conjugates of the present disclosure were evaluated across 10 concentration points, with the highest concentration being 500 nM, in a 4-fold dilution series.

The cells were incubated at 37°C with 5% CO₂ for 48 hours, after which cell morphology was examined under a microscope.

After incubation, the cells were lysed to obtain cell lysates. Total RNA was extract using the RNeasy^{®} 96 Kit, and cDNA was synthesized by reverse transcription using the FastKing RT Kit (With gDNase). INHBE cDNA levels were then measured by qPCR.

4. Experimental conclusion: The conjugates of the present disclosure can significantly reduce INHBE mRNA levels in PHHs.

### Test method 2: In vivo PD study in non-human primates (cynomolgus monkeys)

### 1. Experimental principle:

The degree of reduction in plasma INHBE protein in cynomolgus monkeys induced by the test compounds was evaluated using a non-human primate (cynomolgus monkey) model, which exhibits high homology to the human INHBE gene.

2. Experimental materials: cynomolgus monkeys; 1× PBS (phosphate-buffered saline); conjugates of the present disclosure.

### 3. Experimental procedure:

Ten cynomolgus monkeys weighing 2 to 3 kg were obtained and underwent a one-week acclimatization and quarantine period upon arrival at the animal facility.

Six days prior to dosing, the cynomolgus monkeys were randomly divided into groups based on their body weight, with two animals per group. After grouping, the plasma INHBE protein concentration of each cynomolgus monkey was measured to serve as the baseline value for each group.

Dosing was initiated on Day 1. Blood samples were collected on Days 1, 2, 4, 8, 15, 22, 29, 32, 35, 43, 57, and 71 after dosing, and the plasma INHBE protein concentration of each cynomolgus monkey was measured. The relative reduction in INHBE protein was calculated for each group by comparing the plasma INHBE protein concentration obtained after dosing with the baseline value measured on Day -6.

4. Experimental conclusion: The conjugates of the present disclosure can significantly reduce plasma INHBE protein concentrations in cynomolgus monkeys.

### Test method 3: In vitro RNA sequencing analysis

### 1. Experimental introduction:

The transcriptome refers to the complete collection of RNA molecules transcribed in a particular tissue or cell at a specific time point or under a specific condition, including both mRNAs and non-coding RNAs. Transcriptome sequencing, based on the Illumina sequencing platform, enables analysis of all mRNAs transcribed in a particular tissue or cell at a specific time point. It provides a fundamental tool for research on gene function and structure, and plays a crucial role in understanding the development of organisms and the occurrence of diseases. With the development of sequencing technologies and the reduction of sequencing costs, RNA-seq has become the principal approach for transcriptome analysis owing to its high throughput, high sensitivity, and broad applicability. The RNA-seq workflow mainly includes two stages: library preparation and sequencing, followed by bioinformatics analysis.

### 2. RNA extraction and quality assessment:

RNA is extracted from tissues or cells using standard extraction procedures. The RNA samples are then subjected to rigorous quality control, primarily by using an Agilent 2100 Bioanalyzer to accurately evaluate RNA integrity.

### 3. Library preparation and quality control:

mRNA is isolated by one of the following approaches: (1) enrichment of poly(A)-containing mRNA using Oligo(dT) magnetic beads, taking advantage of the poly(A) tails present on most eukaryotic mRNAs; or (2) removal of ribosomal RNA from total RNA to obtain mRNA. Subsequently, the purified mRNA is randomly fragmented in NEB fragmentation buffer using divalent cations, followed by library preparation according to the standard NEB library preparation protocol or the strand-specific library preparation protocol.

Standard NEB library preparation: Using fragmented mRNA as a template and random oligonucleotides as primers, the first strand of cDNA is synthesized in an M-MuLV reverse transcriptase system. Subsequently, the RNA strand is degraded with RNaseH, and the second strand of cDNA is synthesized using dNTPs as substrates in a DNA polymerase I system. The purified double-stranded cDNA is subjected to end repair, A-tailing, and ligation of sequencing adapters. cDNA fragments of approximately 250 to 300 bp are selected using AMPure XP beads, followed by PCR amplification. The PCR products are purified again using AMPure XP beads to generate the library. Library preparation is performed using the NEBNext^{®} Ultra^{™} RNA Library Prep Kit for Illumina^{®}.

Strand-specific library preparation: The first strand of cDNA is synthesized by reverse transcription as the standard NEB library preparation. The difference lies in the synthesis of the second strand, where dTTP in the dNTPs is replaced by dUTP. Following end repair, A-tailing, ligation of sequencing adapters, and size selection, the U-containing second strand of cDNA is degraded using the USER enzyme, followed by PCR amplification to generate the library. Strand-specific libraries offer numerous advantages, such as generating more effective information with the same amount of data; achieving more accurate gene quantification, localization, and annotation; and providing antisense transcripts and expression levels of individual exons within each transcript isoform. Library preparation is performed using the NEBNext^{®} Ultra^{™} Directional RNA Library Prep Kit for Illumina^{®}.

Note: Sequencing adapters comprise three components: P5/P7, index, and Rd1/Rd2 SP. Specifically, P5/P7 serve as PCR primer binding sites and flow cell attachment sequences; the index sequence enables discrimination among different libraries; Rd1/Rd2 SP represent the read1/read2 sequencing primer binding sites from which sequencing is initiated.

Following library preparation, the library is preliminarily quantified using a Qubit2.0 Fluorometer and diluted to 1.5 ng/µL. Subsequently, the insert size of the library is analyzed using an Agilent 2100 Bioanalyzer. After confirmation of the expected insert size, the effective library concentration is accurately quantified by qRT-PCR (an effective concentration greater than 2 nM) to ensure library quality.

### 4. Sequencing:

Qualified libraries are pooled according to their effective concentrations and the desired sequencing output, followed by Illumina sequencing. The sequencing is based on the principle of sequencing by synthesis. During sequencing, four fluorescently labeled dNTPs, DNA polymerase, and adapter primers are added to the flow cell for amplification. As each fluorescently labeled dNTP is incorporated into the growing complementary DNA strand within a sequencing cluster, a corresponding fluorescence signal is emitted. The sequencer captures these fluorescent signals, which are converted into sequencing reads via computer software, thereby obtaining the sequence information of each target fragment.

5. Experimental conclusion: The conjugates of the present disclosure can significantly reduce INHBE gene expression in RNA sequencing analysis.

### Test method 4: Huh7 cell transfection assay

**1. Experimental objective:** The objective of this study was to evaluate the inhibitory effects of the conjugates described herein on the target gene in Huh7 cells. The inhibitory activity against the target gene INHBE was evaluated using the IC₅₀ value of each compound as an indicator.

### 2. Experimental materials:

**2.1 Cell line:** Huh7 cells; conjugates of the present disclosure.

Huh7 cells were cultured in DMEM containing 10% fetal bovine serum and 1% penicillin-streptomycin.

### 2.2 Reagents:

The principal reagents used in this study include Lipofectamine^{™} RNAiMAX transfection reagent, FastStart Universal Probe Master, RNA extraction kit, GAPDH gene expression assay kit, INHBE gene expression assay kit, FastKing first-strand cDNA synthesis Kit, and 96-well plates.

### 2.3 Consumables and instruments:

The principal instruments used in this study include QuantStudio 6 Flex real-time PCR system (Applied Biosystems) and Vi-cell^{™} XR cell viability analyzer.

### 3. Experimental procedure and methods:

### 3.1 Transfection

1) On Day 1, RNAiMAX transfection reagent was mixed with Opti-MEM, followed by incubation at room temperature for 15 minutes.
2) For each well of cells, an appropriate amount of diluted compound was mixed with an equal volume of the RNAiMAX/Opti-MEM mixture, followed by incubation for 15 minutes.
3) Huh7 cells were washed with DPBS, then trypsinized, and adjusted to an appropriate density.
4) Simultaneously with cell seeding, 20 µL of the Opti-MEM/RNAiMax/compound mixture was added to each well of the culture plate. The cells were seeded into the 96-well plate at a density of 20,000 cells per well, with a final culture volume of 120 µL per well.
5) The cells were incubated at 37°C with 5% CO₂ for 24 hours.

### 3.2 RNA extraction and reverse transcription

Cells were harvested, and total RNA was extracted according to the instructions of the extraction kit. The extracted RNA was reverse-transcribed into cDNA according to the instructions of the FastKing first-strand cDNA synthesis kit.

### 3.3 qPCR analysis

Target gene cDNA was quantified by qPCR using GAPDH as the internal reference gene. qPCR was performed in a 384-well plate according to the following program: 95°C for 10 minutes, followed by 40 cycles of 95°C for 15 seconds and 60°C for 1 minute.

### 3.4 Data analysis: ΔΔCt method (comparative Ct method)

The method requires the introduction of the internal reference gene GAPDH as it is constitutively expressed in all cells, is essential for cell survival, exhibits a constant expression level or genomic copy number, and is minimally affected by environmental conditions. Following qRT-PCR, the CT value of the reference gene was recorded as Ct (GAPDH), and the CT value of the sample was recorded as Ct (sample).$ΔCt \left(sample\right) = Ct \left(sample\right) - Ct \left(GAPDH\right) ;$$ΔCt \left(control\right) = Ct \left(control\right) - Ct \left(GAPDH\right) ;$$ΔΔCt = ΔCt \left(sample\right) - ΔCt \left(control\right) ;$$Relative gene expression = {2}^{- ΔΔCt} .$

4. Experimental conclusion: The conjugates of the present disclosure exhibits effective knockdown of INHBE mRNA in Huh7 cells.

### Technical effects

The double-stranded siRNAs and conjugates of the present disclosure can significantly inhibit INHBE mRNA expression both *in vitro* and *in vivo*, exhibit a low risk of off-target effects and good stability, and are therefore useful for the treatment of diseases associated with the INHBE gene and protein.

### Definitions and description

Unless otherwise specified, the following terms and phrases used herein are intended to have the meanings set forth below. A particular term or phrase should not be considered indefinite or unclear in the absence of an explicit definition, but rather should be interpreted according to the meaning commonly understood by a person of ordinary skill in the art. When a trade name appears herein, it is intended to refer to the corresponding commercial product or the active ingredient thereof.

Unless otherwise specified, the terms "comprising", "including", and "containing" or equivalents thereof are open-ended expressions, meaning that in addition to the listed elements, components, or steps, other unspecified elements, components, or steps may also be encompassed.

Unless otherwise specified, the term "sequence" or "nucleotide sequence" as used herein refers to the order or arrangement of nucleobases or nucleotides represented by a string of letters using standard nucleotide nomenclature.

The term "double-stranded siRNA" refers to a complex of ribonucleic acid molecules having a duplex structure, comprising two antiparallel and substantially complementary nucleotide strands, which have "sense" and "antisense" orientations relative to the target RNA. In the present disclosure, "complementary" has the meaning well known to those skilled in the art, namely that, in a double-stranded nucleic acid molecule, a base on one strand pairs with a base on the other strand in a complementary manner. The purine base adenine (A) pairs with the pyrimidine base uracil (U) or thymine (T), while the purine base guanine (C) pairs with the pyrimidine base cytosine (G). Each base pair consists of one purine and one pyrimidine. Two strands are considered complementary when adenine on one strand pairs with uracil on the other strand and guanine pairs with cytosine, such that the sequence of one strand can be inferred from the sequence of its complementary strand.

The term "single-stranded oligonucleotide" as used herein refers to a single-stranded oligonucleotide having a sequence that is at least partially complementary to a target mRNA and capable of hybridizing to the target mRNA via hydrogen bonding under mammalian physiological conditions (or an equivalent *in vitro* environment). In some embodiments of the present disclosure, the single-stranded oligonucleotide is a single-stranded antisense oligonucleotide.

The term "double-stranded oligonucleotide" as used herein refers to a duplex structure comprising two antiparallel and substantially complementary nucleotide strands, wherein one strand is a sense strand and the other strand is an antisense strand, wherein the antisense strand refers to a strand that is substantially complementary to the corresponding region of a target sequence (e.g., INHBE mRNA) and capable of hybridizing to the target mRNA via hydrogen bonding under mammalian physiological conditions (or an equivalent *in vitro* environment). The term "substantially complementary" means that the corresponding positions of two sequences may be fully complementary or may contain one or more mismatches; when mismatches are present, the sequences typically contain no more than 3, 2, or 1 mismatched base pairs. In a double-stranded nucleic acid molecule, a base on one strand pairs with a base on the other strand in a complementary manner. The purine base adenine (A) pairs with the pyrimidine base uracil (U), while the purine base guanine (C) pairs with the pyrimidine base cytosine (G). In some embodiments of the present disclosure, the double-stranded oligonucleotide is a double-stranded siRNA.

The term "short interfering RNA (siRNA)" as used herein refers to a class of RNA molecules having a duplex region of 17 to 25 base pairs in length, structurally similar to miRNA, that function within the RNA interference (RNAi) pathway. siRNAs interfere with the translation of mRNA corresponding to a complementary nucleotide sequence, thereby inducing degradation of the target mRNA. The siRNAs as used herein include both double-stranded siRNAs (comprising a sense strand and an antisense strand) and single-stranded siRNAs (comprising only an antisense strand).

Unless otherwise specified, the uppercase letters T, C, G, U, and A as used herein represent the base composition of nucleotides.

The term "modification" of a nucleotide as used herein includes, but is not limited to, 2'-O-methyl modification, 2'-fluoro modification, (E)-vinylphosphonate modification, phosphorothioate linkage, or replacement of the nucleotide with a GNA (glycol nucleic acid) monomer, wherein the GNA includes GNA-A, GNA-T, GNA-C, GNA-G, and GNA-U. The sequences described in the present disclosure may include those listed as "further modified sequences" in Table 2.

The 2'-fluoro-modified nucleotide as used herein refers to a nucleotide in which the 2'-hydroxyl group of the ribose moiety is replaced by fluorine. The 2'-O-methyl-modified nucleotide as used herein refers to a nucleotide in which the 2'-hydroxyl group of the ribose moiety is replaced by a methoxy group.

The GNA (glycol nucleic acid) monomers (GNA-A, GNA-T, GNA-C, GNA-G, and GNA-U) as used herein have the following structures:

GNA-A has the structure of 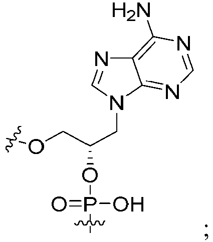 GNA-T has the structure of 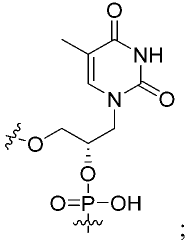 GNA-C has the structure of 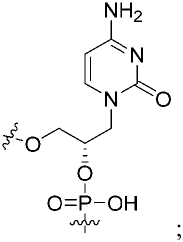 GNA-G has the structure of 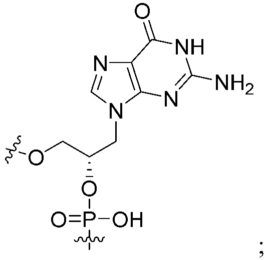 GNA-U has the structure of

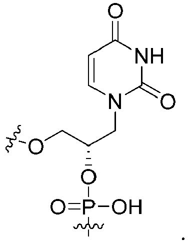

Unless otherwise specified, the phrase "the nucleotide is optionally modified" as used herein means that the nucleotide may be either an unmodified nucleotide or a modified nucleotide. The term "unmodified nucleotide" refers to a nucleotide comprising a natural nucleobase, a natural sugar ring, and a natural phosphate group. The term "modified nucleotide" refers to a nucleotide comprising a modified nucleobase, and/or a modified sugar ring, and/or a modified phosphate group. In some embodiments of the present disclosure, the "modified nucleotide" comprises a modified nucleobase, a modified sugar ring, and a natural phosphate group. In some embodiments of the present disclosure, the "modified nucleotide" comprises a modified nucleobase, a modified phosphate group, and a natural sugar ring. In some embodiments of the present disclosure, the "modified nucleotide" comprises a natural nucleobase, a modified sugar ring, and a modified phosphate group. In some embodiments of the present disclosure, the "modified nucleotide" comprises a modified nucleobase, a natural sugar ring, and a natural phosphate group. In some embodiments of the present disclosure, the "modified nucleotide" comprises a natural nucleobase, a modified sugar ring, and a natural phosphate group. In some embodiments of the present disclosure, the "modified nucleotide" comprises a natural nucleobase, a natural sugar ring, and a modified phosphate group. In some embodiments of the present disclosure, the "modified nucleotide" comprises a modified nucleobase, a modified sugar ring, and a modified phosphate group.

Unless otherwise specified, the term "natural sugar ring" as used herein refers to a 5-membered sugar ring containing a 2'-OH group.

Unless otherwise specified, the term "natural nucleobase" as used herein refers to the purine bases adenine (A) and guanine (G), and the pyrimidine bases thymine (T), cytosine (C), and uracil (U).

Unless otherwise specified, the term "modified nucleobase" as used herein refers to a 5- to 12-membered saturated, partially unsaturated, or aromatic heterocycle other than natural nucleobases, including monocyclic or fused ring systems. Representative examples include, but are not limited to, thiophene, thianthrene, furan, pyran, isobenzofuran, benzothiazine, pyrrole, imidazole, substituted or unsubstituted triazole, pyrazole, isothiazole, isoxazole, pyridazine, indolizine, indole, isoindole, isoquinoline, quinoline, naphthyridine, quinazoline, carbazole, phenanthridine, piperidine, phenazine, phenarsazine, phenothiazine, tetrahydrofuran, phenoxazine, pyrrolidine, pyrroline, imidazolidine, imidazoline, pyrazolidine, 5-methylcytosine (5-me-C), 5-hydroxymethylcytosine, xanthine, hypoxanthine, 2-aminoadenine, 2-aminoadenine, 2-aminoguanine, 2-propyl-substituted adenine and guanine and other alkyl derivatives, 2-thiouracil, 2-thiothymine, 2-thiocytosine, 5-halouracil/halocytosine, 5-propynyluracil/propargylcytosine, 6-azauracil, 6-azacytosine, 6-azathymine, 5-uracil (pseudouracil), 4-thiouracil, 8-halo-, 8-amino-, 8-mercapto-, 8-thioalkyl-, 8-hydroxy- and other 8-substituted adenine and guanine, 5-halo- (particularly 5-bromo-), 5-trifluoromethyl- and other 5-substituted uracil and cytosine, 7-methylguanine, 7-methyladenine, 8-azaguanine, 8-azaadenine, 7-deazaguanine, 7-deazaadenine, 3-deazaguanine, 3-deazaadenine,

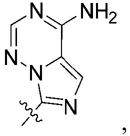

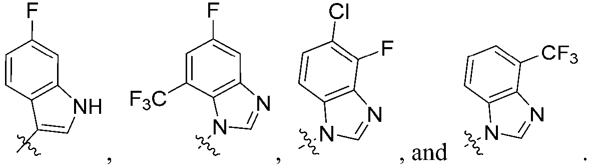

Unless otherwise specified, the term "modified sugar ring" as used herein refers to a sugar ring containing, at the 2' position, one or more modifications selected from, but not limited to: H; F; O-, S-, or N-alkyl; O-, S-, or N-alkenyl; O-, S-, or N-alkynyl; or O-alkyl-O-alkyl, wherein the alkyl, alkenyl, and alkynyl may be substituted or unsubstituted C₁-C₁₀ alkyl, C₂-C₁₀ alkenyl, and C₂-C₁₀ alkynyl. Examples of suitable modifications include O[(CH₂)ₙO]ₘCH₃, O(CH₂)ₙOCH₃, O(CH₂)ₙNH₂, O(CH₂)ₙCH₃, O(CH₂)ₙONH₂, and O(CH₂)ₙON[(CH₂)ₙCH₃)]₂, wherein n and m are integers from 1 to 10. In other embodiments, the modification at the 2' position is selected from, but is not limited to: substituted or unsubstituted C₁-C₁₀ lower alkyl, alkaryl, aralkyl, O-alkaryl, or O-aralkyl; SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, RNA-cleaving groups, reporter groups, intercalating groups, groups for improving the pharmacokinetic properties of iRNA, groups for improving the pharmacodynamic properties of iRNA, and other substituents having similar properties. In some embodiments, the modification includes, but is not limited to, 2'-methoxyethoxy (2'-O-CH₂CH₂OCH₃, also known as 2'-O-(2-methoxyethyl) or 2'-MOE).

Unless otherwise specified, the term "modified phosphate group" as used herein includes, but is not limited to, phosphorothioate modifications, wherein the "phosphorothioate" includes (R)- and (S)-isomers and/or mixtures thereof.

Unless otherwise specified, the term "phosphate" is used herein according to its ordinary meaning as understood by those skilled in the art, and includes its protonated forms (*e.g*., 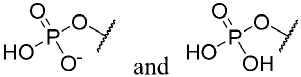 ).

Unless otherwise specified, the terms "phosphorothioate" and "phosphorothioate linkage" refer to compounds of formula 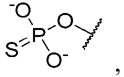 protonated forms thereof (*e.g.*, 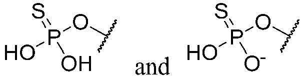 ), and tautomers thereof (*e.g*., 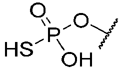 ).

In some embodiments of the present disclosure, the modified nucleotide comprises one or more dX (deoxynucleotide) monomers. The dX monomer includes dA, dT, dC, and dG. dA has the structure of 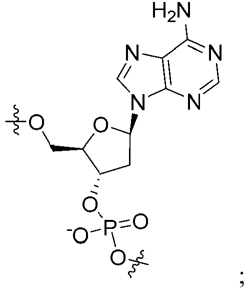 dT has the structure of 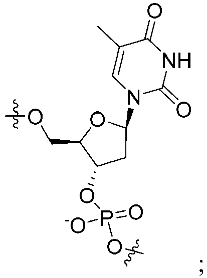 dC has the structure of 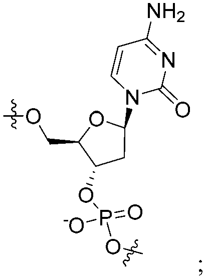 dG has the structure of

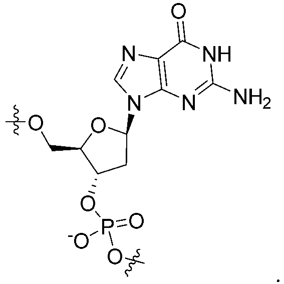

Unless otherwise specified, the term "overhang" as used herein refers to at least one unpaired nucleotide extending from the duplex region of a double-stranded compound. For example, one strand extends beyond the 5' end of the opposite strand at its 3' end, or one strand extends beyond the 3' end of opposite strand at its the 5' end. The overhang may comprise at least one nucleotide, or at least two, three, four, five, or more nucleotides. The nucleotides constituting the overhang are optionally modified nucleotides. The overhang may be present on the sense strand, the antisense strand, or any combination thereof. Furthermore, the overhang may be present at the 5' end, the 3' end, or both ends of either the antisense strand or the sense strand of the double-stranded compound. In some embodiments of the present disclosure, the antisense strand comprises an overhang of 1 to 10 nucleotides (*e.g*., 1, 2, 3, 4, 5, 6, 7, 8, 9, or 10 nucleotides) at the 3' end and/or the 5' end. In some embodiments of the present disclosure, the sense strand comprises an overhang of 1 to 10 nucleotides (*e.g*., 1, 2, 3, 4, 5, 6, 7, 8, 9, or 10 nucleotides) at the 3' end and/or the 5' end. In some embodiments of the present disclosure, both the antisense strand and the sense strand comprise an overhang of 1 to 10 nucleotides (*e.g.,* 1, 2, 3, 4, 5, 6, 7, 8, 9, or 10 nucleotides) at their respective 3' ends. In some embodiments of the present disclosure, both the antisense strand and the sense strand comprise an overhang of 1 to 10 nucleotides (*e.g*., 1, 2, 3, 4, 5, 6, 7, 8, 9, or 10 nucleotides) at their respective 5' ends.

In the present disclosure, a conjugate of a double-stranded siRNA (also referred to simply as a "conjugate") refers to a compound formed by covalently linking the double-stranded siRNA to a pharmaceutically acceptable conjugate moiety.

In the present disclosure, the pharmaceutically acceptable conjugate moiety includes, but is not limited to, antibodies, polypeptides, and ligands.

In the present disclosure, the conjugate comprises 1, 2, 3, 4, or 5 pharmaceutically acceptable conjugate moieties, each of which may be independently attached to any nucleotide of the sense strand and/or the antisense strand of the double-stranded siRNA.

In the present disclosure, the pharmaceutically acceptable conjugate moiety may be attached to the 3' end and/or the 5' end of the sense strand and/or the antisense strand of the double-stranded siRNA.

In the present disclosure, the conjugate comprises 1, 2, 3, 4, or 5 pharmaceutically acceptable conjugate moieties, each of which may be independently attached to the 3' end and/or the 5' end of the sense strand and/or the antisense strand of the double-stranded siRNA.

In the context of the present disclosure, unless otherwise specified, the term "conjugation" refers to the covalent linking of two or more chemical moieties, each having a specific function. Accordingly, the term "conjugate" refers to a compound formed by the covalent linking of such chemical moieties.

In the present disclosure, unless otherwise specified, the term "linker" refers to an organic moiety connecting two portions of a compound, for example, by covalent attachment. The linker typically comprises a direct bond or an atom (*e.g*., oxygen or sulfur), an atomic group (*e.g.,* NRR, C(O), C(O)NH, SO, SO₂, or SO₂NH), substituted or unsubstituted alkyl, substituted or unsubstituted alkenyl, substituted or unsubstituted alkynyl, substituted or unsubstituted aryl, substituted or unsubstituted heteroaryl, substituted or unsubstituted cycloalkyl, or substituted or unsubstituted heterocycloalkyl, wherein one or more carbon atoms in the substituted or unsubstituted alkyl, substituted or unsubstituted alkenyl, or substituted or unsubstituted alkynyl may optionally be replaced by substituted or unsubstituted aryl, substituted or unsubstituted heteroaryl, substituted or unsubstituted cycloalkyl, or substituted or unsubstituted heterocycloalkyl.

A cleavable linker possesses sufficient stability under extracellular conditions but is cleaved upon entering the target cell, thereby releasing the two moieties that are jointly fixed by the linker.

The compounds of the present disclosure may exist in specific geometric or stereoisomeric forms. The present disclosure contemplates all such compounds, including (*R*)- and (*S*)-enantiomers, diastereomers, racemic mixtures, and other mixtures, such as enantiomerically or diastereomerically enriched mixtures, all of which are within the scope of the present disclosure. Additional asymmetric carbon atoms may be present in substituents such as alkyl groups. All such isomers and mixtures thereof are included within the scope of the present disclosure.

Unless otherwise specified, the term "enantiomer" or "optical isomer" refers to stereoisomers that are mirror images of one another.

Unless otherwise specified, the term "diastereomer" refers to stereoisomers containing two or more chiral centers that are non-mirror images of one another.

Unless otherwise specified, a solid wedge bond ( 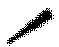 ) and a dashed wedge bond ( 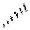 ) indicate the absolute configuration of a stereocenter; a solid straight bond ( 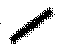 ) and a dashed straight bond ( 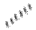 ) indicate the relative configuration of a stereocenter; a wavy line ( 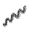 ) represents a solid wedge bond ( 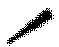 ) or a dashed wedge bond ( 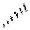 ); alternatively, a wavy line ( 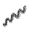 ) represents a solid straight bond ( 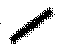 ) or a dashed straight bond ( 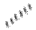 ).

Unless otherwise specified, the terms "enriched in one isomer", "isomer-enriched", "enriched in one enantiomer", or "enantiomer-enriched" mean that one isomer or enantiomer is present at an amount of less than 100%, but greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomeric excess" or "enantiomeric excess" refers to the difference between the relative percentages of two isomers or two enantiomers. For example, if one isomer or enantiomer is present at 90% and the other isomer or enantiomer is present at 10%, the isomeric or enantiomeric excess (ee value) is 80%.

Optically active (*R*)- and (*S*)-isomers, as well as *D*- and *L*-isomers, may be prepared using chiral synthesis, chiral reagents, or other conventional techniques. If a single enantiomer of a compound of the present disclosure is desired, it may be prepared by asymmetric synthesis or derivatization with a chiral auxiliary, in which the resulting diastereomeric mixture is separated and the auxiliary group is cleaved to afford the desired pure enantiomer. Alternatively, when a molecule contains a basic functional group (*e.g.,* an amino group) or an acidic functional group (*e.g.,* a carboxyl group), diastereomeric salts are formed with an appropriate optically active acid or base, followed by resolution of the diastereomers by conventional methods well known in the art to obtain the pure enantiomer. Furthermore, the separation of enantiomers and diastereomers is typically accomplished using chromatography employing chiral stationary phases, optionally in combination with chemical derivatization (*e.g*., conversion of amines to carbamates).

Unless otherwise specified, when a group has one or more attachment sites, any one or more of such sites of the group may be linked to other groups via a chemical bond. The chemical bond linking the sites to other groups may be represented by a wavy line ( 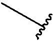 ). For example, the wavy line in 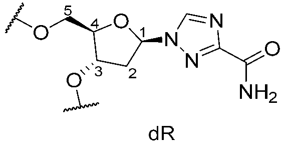 indicates attachment to other nucleotides of the double-stranded siRNA via the 3'- and 5'-oxygen atoms of dR.

The compounds of the present disclosure may contain unnatural proportions of atomic isotopes at one or more of the atoms that constitute such compounds. For example, the compounds may be labeled with radioactive isotopes, such as tritium (³H), iodine-125 (¹²⁵I), or carbon-14 (¹⁴C). For another example, hydrogen atoms may be replaced by deuterium to form deuterated compounds. Since carbon-deuterium bonds are stronger than ordinary carbon-hydrogen bonds, deuterated compounds exhibit advantages over their non-deuterated counterparts, including reduced toxic and side effects, improved drug stability, enhanced therapeutic efficacy, and prolonged biological half-life. All isotopic variants of the compounds of the present disclosure, whether radioactive or non-radioactive, are included within the scope of the present disclosure.

The term "pharmaceutically acceptable salt" as used herein refers to carboxylate salts and amino acid addition salts of the compounds of the present disclosure that are suitable, within the scope of sound medical judgment, for contact with the tissues of a patient without undue toxicity, irritation, or allergic response, and are commensurate with a reasonable benefit/risk ratio in view of their intended use, including (where appropriate) the zwitterionic forms of the compounds of the present disclosure.

The term "salt" refers to salts of the compounds of the present disclosure, prepared by reacting the compounds discovered in the present disclosure, which have specific substituents, with relatively non-toxic acids or bases. When a compound of the present disclosure contains a relatively acidic functional group, a base addition salt may be prepared by contacting the compound with a sufficient amount of an appropriate base in a pure solution or a suitable inert solvent. Pharmaceutically acceptable base addition salts include sodium, potassium, calcium, ammonium, organic amine, and magnesium salts, and the like. When a compound of the present disclosure contains a relatively basic functional group, an acid addition salt may be prepared by contacting the compound with a sufficient amount of an appropriate acid in a pure solution or a suitable inert solvent. Examples of pharmaceutically acceptable acid addition salts include salts derived from inorganic acids, including, but not limited to, hydrochloric acid, hydrobromic acid, nitric acid, carbonic acid, bicarbonate, phosphoric acid, hydrogen phosphate, dihydrogen phosphate, sulfuric acid, hydrogen sulfate, hydroiodic acid, and phosphorous acid; and salts derived from organic acids, including, but not limited to, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, and methanesulfonic acid; as well as salts formed with amino acids (*e.g*., arginine) and salts of organic acids such as glucuronic acid. Certain compounds of the present disclosure contain both basic and acidic functional groups and therefore may be converted into either base or acid addition salts.

The salts of the present disclosure may be synthesized by conventional chemical methods from parent compounds containing an acid moiety or a basic moiety. Generally, such salts are prepared by reacting the free acid or base forms of these compounds with a stoichiometric amount of an appropriate base or acid in water, an organic solvent, or a mixture thereof.

The compounds of the present disclosure may be prepared by a variety of synthetic methods well known to those skilled in the art, including the specific embodiments listed below, embodiments formed by combining such embodiments with other chemical synthesis methods, and equivalent substitutions well known to those skilled in the art. Preferred embodiments include, but are not limited to, the examples of the present disclosure.

The compounds of the present disclosure may contain unnatural proportions of atomic isotopes at one or more of the atoms that constitute such compounds. For example, the compounds may be labeled with radioactive isotopes, such as tritium (³H), iodine-125 (¹²⁵I), or carbon-14 (¹⁴C). For another example, hydrogen atoms may be replaced by deuterium to form deuterated compounds. Since carbon-deuterium bonds are stronger than ordinary carbon-hydrogen bonds, deuterated compounds exhibit advantages over their non-deuterated counterparts, including reduced toxic and side effects, improved drug stability, enhanced therapeutic efficacy, and prolonged biological half-life. All isotopic variants of the compounds of the present disclosure, whether radioactive or non-radioactive, are included within the scope of the present disclosure.

When the direction of attachment of a linking group is not specified, the direction of attachment is arbitrary. For example, the linking group L in 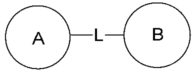 is -M-W-, wherein -M-W- may connect ring A and ring B in the same direction as the reading order from left to right to form 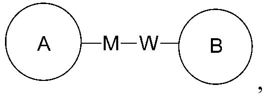 or may connect ring A and ring B in the opposite direction to the reading order from left to right to form 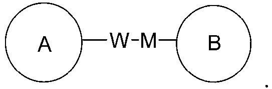 Combinations of the linking groups, substituents, and/or variants thereof are permissible only if such combinations result in chemically stable compounds.

The term "optional" or "optionally" means that the subsequently described event or circumstance may but need not occur, and that the description includes instances in which the event or circumstance occurs and instances in which the event or circumstance does not occur.

The term "substituted" means that any one or more hydrogen atoms attached to a specific atom are replaced by substituents, which may include deuterium and other hydrogen isotopes, provided that the normal valence of the specific atom is maintained and the resulting compound is chemically stable. When the substituent is oxo (*i.e*., =O), two hydrogen atoms are understood to be replaced. Oxygen substitution does not occur on aromatic groups. The term "optionally substituted" means that substitution may or may not occur. Unless otherwise specified, the type and number of substituents may be arbitrary, provided that they are chemically feasible.

When any variable (*e.g*., R) occurs more than once in the composition or structure of a compound, each occurrence is independently defined. Thus, for example, if a group is substituted by 0 to 2 R, the group may be optionally substituted by up to two R, each of which is independently selected. Furthermore, combinations of substituents and/or variants thereof are permissible only if such combinations result in chemically stable compounds.

When the number of a linking group is 0, for example, -(CRR)₀-, the linking group represents a single bond.

When a substituent is indicated as being vacant, it means that the substituent is absent. For example, when X is absent in A-X, the resulting structure is simply A. When the point of attachment of a substituent is not specified, the substituent may be bonded via any atom thereof. For example, a pyridyl group as a substituent may be attached to the substituted group via any carbon atom on the pyridine ring.

The compounds of the present disclosure may be prepared by a variety of synthetic methods well known to those skilled in the art, including the specific embodiments listed below, embodiments formed by combining such embodiments with other chemical synthesis methods, and equivalent substitutions well known to those skilled in the art. Preferred embodiments include, but are not limited to, the examples of the present disclosure.

The structures of the compounds of the present disclosure may be confirmed by conventional methods well known to those skilled in the art. When the present disclosure involves the absolute configuration of a compound, such absolute configuration may be determined by conventional techniques in the art, such as single-crystal X-ray diffraction (SXRD), in which diffraction intensity data is collected from a cultured single crystal using a Bruker D8 Venture diffractometer with CuKα radiation as the X-ray source and φ/ω scanning; after data collection, the crystal structure is further analyzed using the direct method (Shelxs97), thereby confirming the absolute configuration.

Unless otherwise specified, the solvent ratios used in column chromatography and preparative thin-layer silica gel chromatography in the present disclosure are all expressed as volume ratios.

The solvents used in the present disclosure are commercially available.

The following abbreviations are used in the present disclosure: aq represents water; HATU represents O-(7-azabenzotriazol-1-yl)-N,N,N',N'-tetramethyluronium hexafluorophosphate; EDC represents N-(3-dimethylaminopropyl)-N'-ethylcarbodiimide hydrochloride; m-CPBA represents 3-chloroperoxybenzoic acid; eq represents equivalent; CDI represents carbonyldiimidazole; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; Cbz represents benzyloxycarbonyl (amine protecting group); Boc represents *tert*-butoxycarbonyl (amine protecting group); HOAc represents acetic acid; NaCNBH₃ represents sodium cyanoborohydride; r.t. represents room temperature; O/N represents overnight; THF represents tetrahydrofuran; Boc₂O represents di-*tert*-butyl dicarbonate; TFA represents trifluoroacetic acid; DIPEA represents diisopropylethylamine; SOCl₂ represents thionyl chloride; CS₂ represents carbon disulfide; TsOH represents *p*-toluenesulfonic acid; NFSI represents N-fluoro-N-(phenylsulfonyl)benzenesulfonamide; NCS represents 1-chloropyrrolidine-2,5-dione; *n-*Bu₄NF represents tetrabutylammonium fluoride; iPrOH represents 2-propanol; mp represents melting point; LDA represents lithium diisopropylamide; GalNAc moiety represents 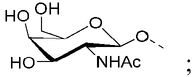 PBS represents phosphate-buffered saline; ASGPR represents asialoglycoprotein receptor, a liver-specific transmembrane glycoprotein.

In some embodiments of the present disclosure, conversion of uppercase letters "A", "U", "C", and "G" to lowercase letters "a", "u", "c", and "g" indicates that the corresponding nucleotide is 2'-O-methyl-modified (a methoxy modification); the suffix "f" following "A", "U", "C", and "G" indicates that the corresponding nucleotide is 2'-fluoro-modified (a fluoro modification); the lowercase letter "s" represents a phosphorothioate linkage between nucleotides; the prefix "d" preceding "A", "U", "C", and "G" indicates that the nucleotide immediately to the right of "d" is a deoxyribonucleotide. For example, the chemical structure represented by 5'-us,Ufs,U,g,dA-3' is as follows:

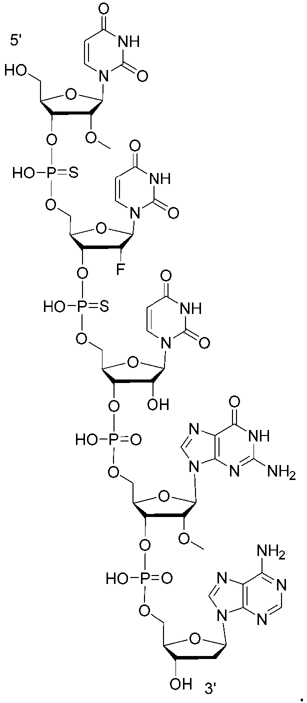

The compounds are named according to conventional nomenclature accepted in the art or using ChemDraw^{®} software. Commercially available compounds are identified by the names used in the corresponding supplier catalogs.

### DETAILED DESCRIPTION OF THE PREFERRED EMBODIMENT

The present disclosure is described in detail below with reference to examples, which do not imply any adverse limitation on the present disclosure. The compounds of the present disclosure may be prepared by a variety of synthetic methods well known to those skilled in the art, including the specific embodiments listed below, embodiments formed by combining such embodiments with other chemical synthesis methods, and equivalent substitutions well known to those skilled in the art. Preferred embodiments include, but are not limited to, the examples of the present disclosure. Various modifications and improvements to the specific embodiments of the present disclosure will be apparent to those skilled in the art without departing from the essence and scope of the present disclosure.

### Example 1: Synthesis of Z1 and Z2

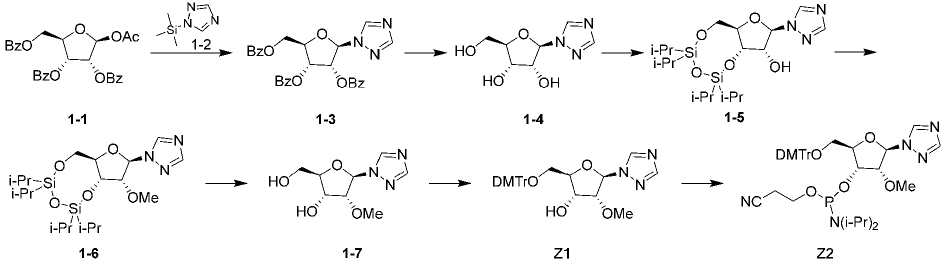

Step A: Compound **1-1** (10 g, 19.82 mmol) was dissolved in acetonitrile (120 mL) and 1,2-dichloroethane (80 mL), followed by addition of compound **1-2** (6.02 g, 42.62 mmol) and trimethylsilyl trifluoromethanesulfonate (11.01 g, 49.56 mmol, 8.95 mL) at 0°C, and the mixture was stirred at 35°C for 12 hours. The reaction mixture was quenched by slow addition of saturated sodium bicarbonate aqueous solution (100 mL) at 0°C and extracted with DCM (100 mL × 2). The organic phase was washed with saturated brine (200 mL), dried over anhydrous sodium sulfate, filtered, and concentrated under reduced pressure to obtain a crude product. The crude product was purified by silica gel column chromatography (eluent: PE/EtOAc = 20:1) to obtain compound **1-3.**

Step B: Compound **1-3** (6.7 g, 13.05 mmol) was dissolved in methanolic ammonia (7 mol/L, 50 mL), and the mixture was stirred at 40°C for 12 hours. The reaction mixture was concentrated under reduced pressure to obtain a crude product. The crude product was purified by silica gel column chromatography (eluent: EtOAc/MeOH = 50/1 to 10/1) to obtain compound **1-4.**

Step C: Compound **1-4** (2.3 g, 11.43 mmol) was dissolved in anhydrous pyridine (25 mL), followed by addition of 1,3-dichloro-1,1,3,3-tetraisopropyldisiloxane (3.64 g, 11.55 mmol, 3.69 mL) at 0°C, and the mixture was stirred at 20°C for 12 hours. The reaction mixture was quenched with water (30 mL) and extracted with EtOAc (30 mL × 2). The organic phase was washed sequentially with hydrochloric acid (1 mol/L, 30 mL × 3) and saturated brine (30 mL), dried over anhydrous sodium sulfate, filtered, and concentrated under reduced pressure to obtain a crude product. The crude product was purified by silica gel column chromatography (eluent: PE/EtOAc = 7/1 to 5/1) to obtain compound **1-5.**

Step D: Compound **1-5** (4 g, 9.02 mmol) was dissolved in acetonitrile (40 mL), followed by sequential addition of silver oxide (8.36 g, 36.06 mmol), 4 Å molecular sieves (3 g), anhydrous pyridine (1.78 g, 22.54 mmol, 1.82 mL), and iodomethane (6.4 g, 45.08 mmol, 2.81 mL), and the mixture was stirred at 25°C for 12 hours. EtOAc (50 mL) was added to the mixture, which was stirred at 20°C for 1 hour. The reaction mixture was filtered through a pad of Celite on a Büchner funnel to collect the filtrate, which added with water (100 mL) and extracted with EtOAc (100 mL × 2). The organic phase was washed with saturated brine (200 mL), dried over anhydrous sodium sulfate, filtered, and concentrated under reduced pressure to obtain a crude product. The crude product was purified by silica gel column chromatography (eluent: PE/EtOAc = 15/1 to 10/1 to 8/1) to obtain compound **1-6.**

Step E: Compound **1-6** (2 g, 4.37 mmol) was dissolved in anhydrous THF (20 mL), followed by addition of triethylamine trihydrofluoride (1.55 g, 9.61 mmol, 1.57 mL), and the mixture was stirred at 20°C for 12 hours. The reaction mixture was concentrated under reduced pressure to obtain a crude product. The crude product was purified by silica gel column chromatography (eluent: EtOAc/MeOH = 100/1 to 50/1) to obtain compound **1-7.**

Step F: Compound **1-7** (1 g, 4.65 mmol) was dissolved in anhydrous pyridine (10 mL), followed by addition of 4,4-dimethoxytrityl chloride (1.57 g, 4.65 mmol), and the mixture was stirred at 20°C for 12 hours. The reaction mixture was quenched with water (20 mL) and extracted with EtOAc (20 mL × 2). The organic phase was washed with saturated brine (40 mL), dried over anhydrous sodium sulfate, filtered, and concentrated under reduced pressure to obtain a crude product. The crude product was purified by silica gel column chromatography (eluent: PE/EtOAc = 3/1 to 1/1) to obtain Z1. ¹H NMR (400 MHz, DMSO-*d*₆)*:* δ = 8.78 (s, 1H), 8.14 - 8.03 (m, 1H), 7.41 - 7.31 (m, 2H), 7.29 - 7.16 (m, 7H), 6.92 - 6.76 (m, 4H), 6.08 (d, *J* = 3.2 Hz, 1H), 5.25 (d, *J =* 6.5 Hz, 1H), 4.48 - 4.34 (m, 1H), 4.13 - 4.01 (m, 2H), 3.73 (s, 6H), 3.38 (s, 3H), 3.18 - 3.03 (m, 2H).

Step G: DCM (5.5 L), Z1 (1.1 g, 2.13 mmol), bis(diisopropylamino)(2-cyanoethoxy)phosphine (0.7 g, 2.34 mmol), and 4,5-dicyanoimidazole (0.13 g, 1.06 mmol) were sequentially added to a 10 L dry reactor at room temperature under a nitrogen atmosphere, and the mixture was stirred at a controlled temperature of 20 to 25°C for 3 to 4 hours. After completion of the reaction, the reaction mixture was quenched with saturated sodium bicarbonate aqueous solution (1 mL) at 20 to 25°C, then diluted with water (10 mL), and allowed to stand for phase separation. The organic phase was washed with water (10 mL), dried over anhydrous sodium sulfate (1 g), filtered, and the filtrate was concentrated under reduced pressure to obtain a crude product. The residue was transferred to a round-bottom flask and dissolved in toluene (15 mL) and n-heptane (10 mL) with stirring. The reaction mixture was washed three times with DMF/water (7/3, 5 mL × 3), followed by three washes with water (5 mL × 3) and one wash with saturated brine (5 mL). The organic phase was dried over anhydrous sodium sulfate (11 g), filtered, concentrated, and co-evaporated with acetonitrile to obtain Z2. ¹H NMR (400 MHz, DMSO-*d*₆) δ = 8.82 (s, 1H), 8.11 (d, *J =* 6.6 Hz, 1H), 7.42 - 7.32 (m, 2H), 7.30 - 7.16 (m, 7H), 6.90 - 6.76 (m, 4H), 6.20 - 6.08 (m, 1H), 4.76 - 4.51 (m, 1H), 4.29 (q, *J* = 4.2 Hz, 1H), 4.23 - 4.09 (m, 1H), 3.83 - 3.75 (m, 1H), 3.72 (d, *J =* 2.4 Hz, 6H), 3.61 - 3.45 (m, 3H), 3.38 (d, *J =* 15.2 Hz, 3H), 3.30 - 3.16 (m, 1H), 3.07 (br dd, *J =* 5.6, 10.4 Hz, 1H), 2.77 (t, *J =* 5.9 Hz, 1H), 2.63 - 2.55 (m, 1H), 1.16 - 1.07 (m, 9H), 0.96 (d, *J =* 6.6 Hz, 3H).

Z2 is an intermediate for the preparation of Z-containing sense strands, antisense strands, or conjugates.

### Example 2: B19-M

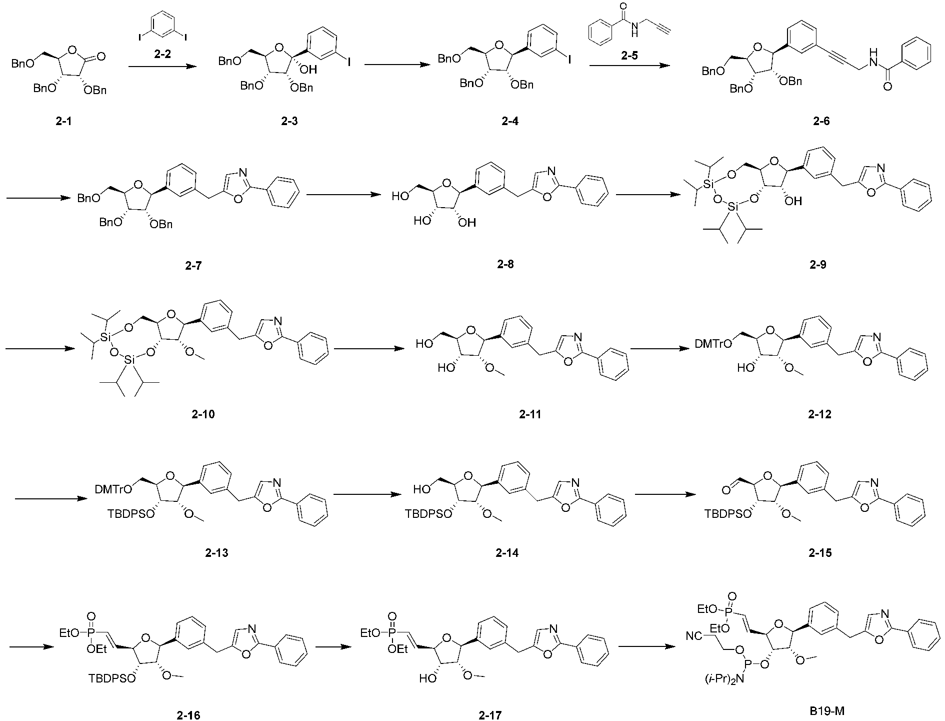

Step 1: Under a nitrogen atmosphere, n-butyllithium (2.5 mol/L, 94.57 mL) was added to a solution of compound **2-2** (78 g, 236.43 mmol) in THF (1000 mL) at -78°C over a period of 10 minutes. After completion of the addition, the mixture was stirred at -78°C for 20 minutes, followed by addition of a solution of compound **2-1** (98.94 g, 236.43 mmol) in THF (500 mL) at -78°C. The mixture was stirred at -78°C for 1.5 hours. The reaction mixture was quenched with saturated ammonium chloride aqueous solution (500 mL) at 0°C and extracted with EtOAc (1000 mL). The organic phase was washed with saturated brine (500 mL × 2), dried over anhydrous sodium sulfate, filtered, and concentrated under reduced pressure to obtain a crude product. The crude product was purified by silica gel column chromatography (PE/EtOAc = 1/0 to 3/1) to obtain compound **2-3.**

Step 2: Under a nitrogen atmosphere, boron trifluoride diethyl etherate (250.80 g, 1.77 mol/L, 217.33 mL) and triethylsilane (205.47 g, 1.77 mol/L) were added to a solution of compound **2-3** (110 g, 176.71 mmol) in DCM (1100 mL) at -78°C, and the mixture was stirred at -78°C for 2 hours. The reaction mixture was quenched with 5% sodium carbonate aqueous solution (500 mL) at 0°C and extracted with DCM (300 mL × 2). The combined organic phases were washed with saturated brine (200 mL × 2), dried over anhydrous sodium sulfate, filtered, and concentrated under reduced pressure to obtain a crude product. The crude product was purified by silica gel column chromatography (PE/EtOAc = 1/0 to 5/1) to obtain compound **2-4.**

Step 3: Under a nitrogen atmosphere, compound **2-4** (73 g, 120.36 mmol), compound **2-5** (21.08 g, 132.40 mmol), cuprous iodide (2.29 g, 12.04 mmol), N,N-diisopropylethylamine (104.83 mL), and Pd(dppf)Cl₂ (8.81 g, 12.04 mmol) were added to acetonitrile (400 mL), and the mixture was stirred at 25°C for 2 hours. The reaction mixture was diluted with water (300 mL) and extracted with EtOAc (500 mL × 2). The combined organic phases were washed with saturated brine (300 mL × 2), dried over anhydrous sodium sulfate, filtered, and concentrated to obtain a crude product. The crude product was purified by silica gel column chromatography (PE/EtOAc = 1/0 to 3/1) to obtain compound **2-6.**

Step 4: Under a nitrogen atmosphere, compound **2-6** (70 g, 109.76 mmol) and cesium carbonate (71.52 g, 219.52 mmol) were added to acetonitrile (700 mL), and the mixture was stirred at 80°C for 16 hours. The reaction mixture was concentrated under reduced pressure to remove acetonitrile, diluted with water (100 mL), and extracted with EtOAc (100 mL × 2). The combined organic phases were washed with saturated brine (50 mL × 2), dried over anhydrous sodium sulfate, filtered, and concentrated under reduced pressure to obtain a crude product. The crude product was purified by silica gel column chromatography (PE/EtOAc = 1/0 to 3/1) to obtain compound **2-7.**

Step 5: Under a nitrogen atmosphere, boron trichloride (1 mol/L, 627.19 mL) was added to a solution of compound **2-7** (50 g, 78.40 mmol) in DCM (500 mL) at -78°C, and the mixture was stirred at -78°C for 2 hours until completion. The reaction mixture was quenched by slow dropwise addition of MeOH (100 mL) at -78°C, and concentrated under reduced pressure to obtain a crude product. The crude product was purified by silica gel column chromatography (PE/EtOAc = 1/0 to 1/1, followed by EtOAc/EtOH = 10/1 to 5/1) to obtain compound **2-8.**

Step 6: Under a nitrogen atmosphere, compound **2-8** (28.8 g, 78.39 mmol), 1,3-dichloro-1,1,3,3-tetraisopropyldisiloxane (29.67 g, 94.07 mmol), and imidazole (21.35 g, 313.56 mmol) were added to DCM (280 mL), and the mixture was stirred at 25°C for 16 hours. The reaction mixture was diluted with water (200 mL) and extracted with DCM (200 mL × 2). The combined organic phases were washed with saturated brine (200 mL), dried over anhydrous sodium sulfate, filtered, and concentrated under reduced pressure to obtain a crude product. The crude product was purified by silica gel column chromatography (PE/EtOAc = 1/0 to 4/1) to obtain compound **2-9.**

Step 7: Under a nitrogen atmosphere, sodium hydride (2.20 g, 55.09 mmol, 60% purity) was slowly added to a solution of compound **2-9** (28 g, 45.91 mmol) in DMF (140 mL) at 0°C. After completion of the addition, the mixture was stirred at 0°C for 0.5 hours, followed by addition of iodomethane (8.57 mL), and stirred at 25°C for 1.5 hours. The reaction mixture was quenched with saturated ammonium chloride aqueous solution (100 mL) at 10 to 25°C, diluted with water (200 mL), and extracted with EtOAc (200 mL × 2). The combined organic phases were washed with saturated brine (30 mL × 2), dried over anhydrous sodium sulfate, filtered, and concentrated under reduced pressure to obtain a crude product. The crude product was purified by silica gel column chromatography (PE/EtOAc = 1/0 to 4/1) to obtain compound **2-10.**

Step 8: Under a nitrogen atmosphere, compound **2-10** (22.3 g, 35.74 mmol) and triethylamine hydrofluoride (9.53 g, 78.63 mmol) were added to THF (160 mL), and the mixture was stirred at 25°C for 16 hours. The reaction mixture was concentrated under reduced pressure to obtain a crude product. The crude product was purified by silica gel column chromatography (PE/EtOAc = 1/0 to 0/1) to obtain compound **2-11.**

Step 9: Under a nitrogen atmosphere, compound **2-11** (10.6 g, 27.79 mmol), 4,4-dimethoxytrityl chloride (11.30 g, 33.35 mmol), and triethylenediamine (4.68 g, 41.69 mmol) were mixed in DCM (60 mL), and the mixture was stirred at 25°C for 16 hours. The reaction mixture was diluted with water (30 mL) and extracted with DCM (50 mL × 2). The combined organic phases were washed with saturated brine (20 mL), dried over anhydrous sodium sulfate, filtered, and concentrated under reduced pressure to obtain a crude product. The crude product was purified by silica gel column chromatography (PE/EtOAc = 1/0 to 1/1, 0.1% triethylamine) to obtain compound **2-12.**

Step 10: Under a nitrogen atmosphere, compound **2-12** (17 g, 24.86 mmol), imidazole (3.39 g, 49.72 mmol), and tert-butyldiphenylchlorosilane (8.20 g, 29.83 mmol) were added to DCM (170 mL), and the mixture was stirred at 25°C for 16 hours. The reaction mixture was diluted with water (20 mL) and extracted with DCM (30 mL × 2). The combined organic phases were washed with saturated brine (30 mL), dried over anhydrous sodium sulfate, filtered, and concentrated to obtain a crude product. The crude product was purified by silica gel column chromatography (PE/EtOAc = 1/0 to 2/1) to obtain compound **2-13.**

Step 11: Under a nitrogen atmosphere, compound **2-13** (21 g, 22.77 mmol) and *p-*toluenesulfonyl chloride monohydrate (2.17 g, 11.39 mmol) were added to DCM (200 mL) and MeOH (60 mL), and the mixture was stirred at 5°C for 1 hour. The reaction mixture was quenched with saturated sodium bicarbonate aqueous solution (20 mL) and extracted with DCM (50 mL × 2). The combined organic phases were washed with saturated brine (30 mL × 2), dried over anhydrous sodium sulfate, filtered, and concentrated under reduced pressure to obtain a crude product. The crude product was purified by silica gel column chromatography (PE/EtOAc = 1/0 to 2/1) to obtain compound **2-14.**

Step 12: Under a nitrogen atmosphere, compound **2-14** (13.7 g, 22.10 mmol) and Dess-Martin periodinane (11.25 g, 26.52 mmol) were added to DCM (100 mL), and the mixture was stirred at 25°C for 1 hour. The reaction mixture was quenched with saturated sodium bicarbonate aqueous solution (20 mL) and extracted with DCM (50 mL × 2). The combined organic phases were washed with saturated brine (30 mL × 2), dried over anhydrous sodium sulfate, filtered, and concentrated under reduced pressure to obtain a crude product. The crude product was purified by silica gel column chromatography (PE/EtOAc = 1/0 to 2/1) to obtain compound **2-15.**

Step 13: Under a nitrogen atmosphere, compound **2-15** (2 g, 3.24 mmol), tetraethyl methylenediphosphonate (1.12 g, 3.88 mmol), lithium chloride (274.48 mg, 6.47 mmol), and 1,8-diazabicyclo[5.4.0]undec-7-ene (1.48 g, 9.71 mmol) were added to DCM (20 mL), and the mixture was stirred at 25°C for 3 hours. The reaction mixture was quenched with water (10 mL) at 25°C and extracted with DCM (30 mL × 2). The combined organic phases were washed with saturated brine (30 mL), dried over anhydrous sodium sulfate, filtered, and concentrated under reduced pressure to obtain a crude product. The crude product was purified by silica gel column chromatography (PE/EtOAc = 1/0 to 1/1) to obtain compound **2-16.**

Step 14: Under a nitrogen atmosphere, compound **2-16** (2.1 g, 2.79 mmol) and triethylamine trihydrofluoride (676.97 mg, 5.59 mmol) were added to THF (10 mL), and the mixture was stirred at 40°C for 16 hours. The reaction mixture was concentrated under reduced pressure to obtain a crude product. The crude product was purified by silica gel column chromatography (PE/EtOAc = 1/0 to 0/1, followed by EtOAc/EtOH = 1/0 to 5/1) to obtain compound **2-17.**

Step 15: Under a nitrogen atmosphere, compound **2-17** (1.3 g, 2.53 mmol), bis(diisopropylamino)(2-cyanoethoxy)phosphine (1.07 g, 3.54 mmol), and 4,5-dicyanoimidazole (149.48 mg, 1.27 mmol) were added to DCM (15 mL), and the mixture was stirred at 25°C for 1 hour. The reaction mixture was diluted with water (10 mL) and extracted with DCM (50 mL × 2). The combined organic phases were washed with saturated brine (30 mL × 2), dried over anhydrous sodium sulfate, filtered, and concentrated under reduced pressure to obtain a crude product. The crude product was purified by reverse-phase column chromatography (column: Waters Xbridge C18, 150 × 50 mm × 10 µm; mobile phase: [10 mmol/L ammonium bicarbonate aqueous solution-acetonitrile]; acetonitrile gradient: 57% to 87%) to obtain **B19-M.** ¹H NMR (400 MHz, DMSO-*d*₆) δ = 8.00 - 7.82 (m, 2H), 7.58 - 7.44 (m, 3H), 7.43 - 7.23 (m, 4H), 7.04 (s, 1H), 6.90 - 6.65 (m, 1H), 6.10 (br dd, *J =* 18.0, 19.4 Hz, 1H), 4.89 (dd, *J* = 5.9, 15.8 Hz, 1H), 4.69 - 4.53 (m, 1H), 4.33 - 4.19 (m, 1H), 4.15 (s, 2H), 4.04 - 3.92 (m, 4H), 3.86 - 3.53 (m, 5H), 3.34 - 3.29 (m, 3H), 2.80 (td, *J =* 5.4, 11.0 Hz, 2H), 1.30 - 1.06 (m, 18H).

B19-M is an intermediate for the preparation of B19-containing sense strands, antisense strands, or conjugates.

### Example 3: B33-M

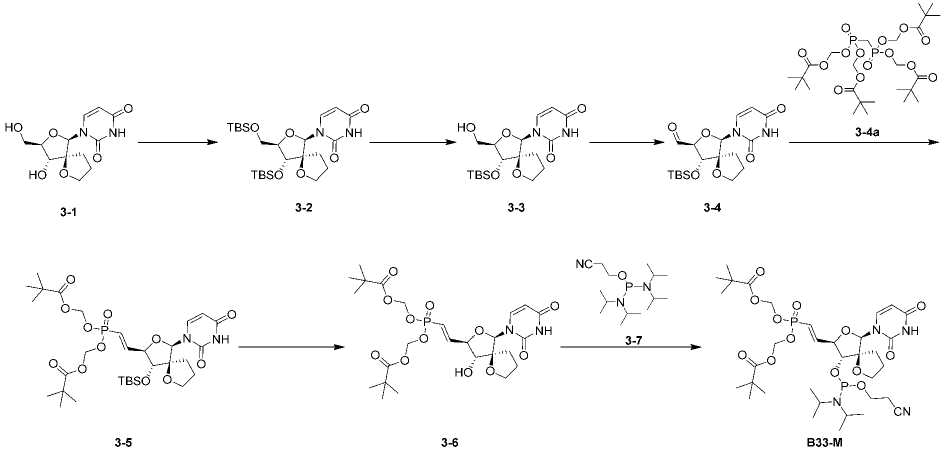

Step 1: Compound **3-1** (1.9 g, 6.68 mmol) was dissolved in DMF (30 mL), followed by addition of imidazole (2.73 g, 40.10 mmol), 4-dimethylaminopyridine (244.97 mg, 2.01 mmol), and tert-butyldimethylchlorosilane (4.03 g, 26.74 mmol), and the mixture was stirred at 65°C for 16 hours. The reaction mixture was diluted with EtOAc (100 mL) and washed with saturated brine (100 mL × 2). The organic phase was dried over anhydrous sodium sulfate, filtered, and concentrated under reduced pressure to obtain a crude product. The crude product was purified by silica gel column chromatography (PE/EtOAc = 1/1 to 1/2) to obtain compound **3-2.** LCMS (ESI) m/z: 513 [M+H]⁺.

Step 2: Compound **3-2** (2.5 g, 4.88 mmol) was dissolved in THF (24 mL), followed by addition of trifluoroacetic acid aqueous solution (24 mL, v/v = 1/1), and the mixture was stirred at 25°C for 2 hours. The reaction mixture was diluted with EtOAc (120 mL) and washed with saturated sodium bicarbonate aqueous solution (120 mL) and water (120 mL). The organic phase was dried over anhydrous sodium sulfate, filtered, and concentrated under reduced pressure to obtain a crude product. The crude product was purified by silica gel column chromatography (PE/EtOAc = 2/1 to 1/1) to obtain compound **3-3.** LCMS (ESI) m/z: 399 [M+H]⁺.

Step 3: Compound **3-3** (1.8 g, 4.52 mmol) was dissolved in acetonitrile (30 mL), followed by addition of 2-iodoxybenzoic acid (2.53 g, 9.03 mmol), and the mixture was stirred at 50°C for 16 hours. The reaction mixture was filtered, and the filtrate was concentrated to obtain crude compound **3-4.** LCMS (ESI) m/z: 397 [M+H]⁺.

Step 4: Under a nitrogen atmosphere, sodium hydride (411.55 mg, 10.29 mmol, 60% purity) and compound **3-4a** (5.42 g, 8.57 mmol) were added to THF (15 mL) at -78°C, and the mixture was stirred at -78°C for 0.5 hours. A solution of compound **3-4** (1.7 g, 4.29 mmol) in THF (15 mL) was then added, and the mixture was stirred at 25°C for 1 hour. The reaction mixture was quenched with saturated ammonium chloride aqueous solution (30 mL) and extracted with EtOAc (30 mL). The organic phase was dried over anhydrous sodium sulfate, filtered, and concentrated under reduced pressure to obtain a crude product. The crude product was purified by silica gel column chromatography (PE/EtOAc = 2/1 to 1/1) to obtain compound **3-5.**

Step 5: Compound **3-5** (1.3 g, 1.85 mmol) was added to formic acid (13 mL) and water (13 mL), and the mixture was stirred at 65°C for 12 hours. The reaction mixture was diluted with EtOAc (120 mL) and washed with saturated sodium bicarbonate aqueous solution (120 mL) and water (120 mL). The organic phase was dried over anhydrous sodium sulfate, filtered, and concentrated under reduced pressure to obtain a crude product. The crude product was purified by silica gel column chromatography (PE/EtOAc = 2/1 to 1/1) to obtain compound **3-6.**

Step 6: Under a nitrogen atmosphere, compound **3-6** (520 mg, 883.54 µmol) was dissolved in DCM (10 mL), followed by addition of 4,5-dicyanoimidazole (520 mg, 883.54 µmol) and compound **3-7** (399.46 mg, 1.33 mmol), and the mixture was stirred at 25°C for 16 hours. The reaction mixture was concentrated under reduced pressure to obtain a crude product. The crude product was purified by high-performance liquid chromatography (column: Waters Xbridge C18, 150 × 50 mm × 10 µm; mobile phase: [10 mmol/L ammonium bicarbonate aqueous solution-acetonitrile]; acetonitrile gradient: 54% to 84%) to obtain compound **B33-M.** ¹H NMR (400 MHz, DMSO-*d*₆) δ = 11.45 (s, 1H), 7.62 - 7.58 (m, 1H), 6.99 - 6.75 (m, 1H), 6.22 - 6.10 (m, 1H), 6.04 (s, 1H), 5.66 - 5.59 (m, 5H), 4.58 - 4.51 (m, 1H), 4.17 - 4.01 (m, 1H), 3.84 - 3.68 (m, 3H), 3.65 - 3.51 (m, 3H), 2.84 - 2.78 (m, 2H), 2.39 - 2.16 (m, 1H), 1.94 - 1.80 (m, 2H), 1.77 - 1.64 (m, 1H), 1.17 (s, 30H).

B33-M is an intermediate for the preparation of B33-containing sense strands, antisense strands, or conjugates.

### Example 3: Preparation method of conjugates

Preparation method: Oligoribonucleotides were synthesized using phosphoramidite solid-phase synthesis technology on a solid support made of controlled pore glass (CPG, 500 Å). All 2'-modified RNA phosphoramidites and auxiliary reagents were commercially available. All amidites were dissolved in anhydrous acetonitrile, followed by addition of molecular sieves (3 Å). Benzyl-1H-tetrazole (BTT) was used as an activator, with a coupling time of 5 minutes. A solution of ((dimethylamino-methylene)amino)-3H-1,2,4-dithiazole-3-thione (DDTT), 1 g/100 mL, in anhydrous acetonitrile/pyridine (v/v = 1/2) was used to generate phosphodiester or phosphorothioate linkages, with a reaction time of 3 minutes. All sequences were synthesized after final removal of the DMT group.

Cleavage and deprotection of CPG-bound oligomers: After completion of solid-phase synthesis, the protecting groups were removed by treatment with 10% diethylamine in acetonitrile for 30 minutes, without cleaving the nucleotides from the CPG. Subsequently, the dried CPG was treated with concentrated ammonia at 40°C for 18 hours. After centrifugation, the supernatant was transferred to a new tube and the CPG was washed with water. The combined solutions were concentrated to obtain a solid mixture.

Purification: Oligomers were purified using NanoQ anion-exchange HPLC. Buffer A consisted of 10 mM sodium perchlorate, 20 mM Tris, 1 mM EDTA, pH 7.4, containing 20% acetonitrile. Buffer B consisted of 500 mM sodium perchlorate, 20 mM Tris, 1 mM EDTA, pH 7.4, containing 20% acetonitrile. The target product was isolated and desalted using a reverse-phase C18 column.

Annealing: RNA oligomers to be annealed were prepared in sterile RNase-free water at 200 µM. Complementary strands were formed by combining equimolar amounts of RNA solutions. The annealing conditions were as follows: The mixture (total volume of 100 µL, 10 nmol) was heated in a water bath at 95°C for 10 minutes, then rapidly transferred to a water bath at 60°C, and allowed to cool naturally. The annealed solution should not be stored at elevated temperatures.

For example, in Examples 4 and 5, the conjugates may be prepared following the method described above, by adjusting the order of starting materials according to the sequences of nucleotides, dR, R, and Z. All conjugates listed in Table 3 were prepared using the method.

### Example 4: Preparation of conjugate C1

Sense strand: 5'-cs,cs,a,g,u,c,g,u,Cf,Cf,Cf,a,g,a,a,u,a,a,c,u,a,D01-3' (SEQ ID NO: 161);
Antisense strand: 5'-us,dAs,g,u,dT,a,dT,u,c,u,g,dG,g,Af,c,g,a,c,u,g,gs,us,c-5' (SEQ ID NO: 189);
wherein D01 is linked to a via a phosphodiester bond.

### Example 5: Preparation of conjugate C25

Sense strand: 5'-cs,cs,a,g,u,c,g,u,Cf,Cf,Cf,a,g,a,a,u,a,a,c,u,a,D01-3' (SEQ ID NO: 161);
Antisense strand: 5'-us,dAs,g,u,dT,a,dR,u,c,u,g,dG,g,Af,c,g,a,c,R,g,gs,us,c-3' (SEQ ID NO: 210);
wherein D01 is linked to a via a phosphodiester bond.

### Biological evaluation

### Test Example 1: Inhibition of INHBE mRNA expression in Huh7 cells

**1. Experimental objective:** The objective of this study was to evaluate the *in vitro* inhibitory activity of siRNAs against the target gene INHBE mRNA in Huh7 cells. The inhibitory activity against INHBE mRNA was evaluated using the Eₘₐₓ value of each compound as an indicator.

### 2. Experimental materials:

### 2.1 Cell line: Huh7 cells.

Huh7 cells were provided by WuXi AppTec Co., Ltd. Huh7 cells were cultured in DMEM (Invitrogen, Cat. No. 11965118) containing 10% fetal bovine serum (ExCellBio, Cat. No. FSP500) and 1% penicillin-streptomycin (HyClone, Cat. No. SV30010).

### 2.2 Reagents:

The principal reagents used in this study include Lipofectamine^{™} RNAiMAX transfection reagent, FastStart Universal Probe Master, RNA extraction kit, GAPDH gene expression assay kit, INHBE gene expression assay kit, FastKing first-strand cDNA synthesis Kit, and 96-well plates.

### 2.3 Consumables and instruments:

The principal instruments used in this study include QuantStudio 6 Flex real-time PCR system (Applied Biosystems) and Vi-cell^{™} XR cell viability analyzer.

### 3. Experimental procedure and methods:

### 3.1 Transfection

1) On Day 1, a transfection mixture was prepared by mixing RNAiMAX transfection reagent with Opti-MEM at a ratio of 1.5:48.5, followed by incubation at room temperature for 15 minutes.
2) For each well of cells, an appropriate amount of diluted compound was mixed with an equal volume of the RNAiMAX/Opti-MEM mixture, followed by incubation for 15 minutes.
3) Huh7 cells were washed with DPBS, then trypsinized, and adjusted to an appropriate density.
4) Simultaneously with cell seeding, 20 µL of the Opti-MEM/RNAiMax/compound mixture was added to each well of the culture plate. The cells were seeded into the 96-well plate at a density of 20,000 cells per well, with a final culture volume of 120 µL per well.
5) The cells were incubated at 37°C with 5% CO₂ for 24 hours.

### 3.2 RNA extraction and reverse transcription

Cells were harvested, and total RNA was extracted according to the instructions of the RNA extraction kit (QIAGEN, Cat. No. 74182). The extracted RNA was reverse-transcribed into cDNA according to the instructions of the FastKing first-strand cDNA synthesis kit.

### 3.3 qPCR analysis

Target gene cDNA was quantified by qPCR. The qPCR reaction system is shown in Table 4. GAPDH was used as the internal reference gene. qPCR was performed in a 384-well plate according to the following program: 95°C for 10 minutes, followed by 40 cycles of 95°C for 15 seconds and 60°C for 1 minute.

**Table 4: RT-PCR reaction system**

| **Component** | **Concentration** | **Volume (µL) per well** |
|---|---|---|
| PCR Master Mix | 2× | 5 |
| INHBE/GAPDH TaqMan^{®} Assays and Arrays (60×) | 60× | 0.17 |
| Water | / | 2.83 |
| Total | / | 8 |

### 3.4 Data analysis: ΔΔCt method (comparative Ct method)

The method requires the introduction of the housekeeping gene GAPDH as it is constitutively expressed in all cells, is essential for cell survival, exhibits a constant expression level or genomic copy number, and is minimally affected by environmental conditions. Following qRT-PCR, the CT value of the reference gene was recorded as Ct (GAPDH), and the CT value of the sample was recorded as Ct (sample).$ΔCt \left(sample\right) = Ct \left(sample\right) - Ct \left(GAPDH\right) ;$$ΔCt \left(control\right) = Ct \left(control\right) - Ct \left(GAPDH\right) ;$$ΔΔCt = ΔCt \left(sample\right) - ΔCt \left(control\right) ;$$Relative gene expression = {2}^{- ΔΔCt} ;$ 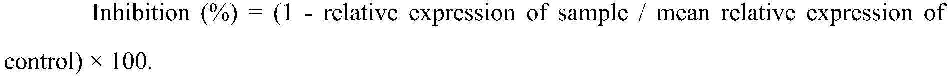 Inhibition (%) = (1 - relative expression of sample / mean relative expression of control) × 100.

4. The experimental results are shown in Table 5.

**Table 5: Inhibitory activity against INHBE mRNA expression in Huh7 cells by conjugates of the present disclosure**

| Conjugate | IC₅₀ (nM) | Maximum inhibition (%) |
|---|---|---|
| C1 | 0.07 | 84.1 |
| C4 | 0.68 | 87.3 |
| C5 | 0.06 | 76.8 |
| C11 | 0.12 | 86.3 |
| C12 | 0.08 | 82.7 |
| C14 | 0.05 | 81.5 |
| C23 | 0.05 | 86.4 |
| C25 | 0.06 | 87.8 |
| C27 | 0.66 | 63.9 |
| C30 | 0.16 | 80.1 |
| C42 | 0.13 | 85.8 |
| C43 | 0.18 | 78.71 |
| C44 | 0.28 | 78.6 |
| C45 | 0.13 | 83.8 |
| C47 | 0.12 | 81.91 |

5. Conclusion: The conjugates of the present disclosure can significantly reduce INHBE mRNA levels in Huh7 cells.

### Test Example 2: Free uptake assay in primary human hepatocytes (PHHs)

### 1. Experimental principle:

The degree of reduction in INHBE mRNA levels induced by the test compounds was evaluated by incubating the test compounds with PHHs.

### 2. Experimental materials:

Primary human hepatocytes (PHHs); RNeasy^{®} 96 Kit (12) (QIAGEN, Cat. No. 74182); FastKing RT Kit (With gDNase) (TIANGEN, Cat. No. KR116-02).

### 3. Experimental procedure:

The conjugates of the present disclosure were diluted with PBS to 10-fold the test concentrations. 10 µL of each siRNA solution was transferred to a 96-well plate. PHHs were thawed and seeded into the 96-well plate at a final density of 5.4 × 10⁴ cells/well. The conjugates of the present disclosure were evaluated across 10 concentration points, with the highest concentration being 500 nM, in a 5-fold dilution series.

The cells were incubated at 37°C with 5% CO₂ for 48 hours, after which cell morphology was examined under a microscope.

After incubation, the cells were lysed to obtain cell lysates. Total RNA was extract using the RNeasy^{®} 96 Kit (QIAGEN, Cat. No. 74182), and cDNA was synthesized by reverse transcription using the FastKing RT Kit (With gDNase) (TIANGEN, Cat. No. KR116-02). INHBE cDNA levels were then measured by qPCR.

4. The experimental results are shown in Table 6.

**Table 6: Results of free uptake assay of conjugates of the present disclosure in PHHs**

| Conjugate | IC₅₀ (nM) | Maximum inhibition (%) |
|---|---|---|
| C25 | 0.49 | 88.0 |
| C42 | 0.18 | 74.1 |
| C43 | 0.13 | 79.9 |
| C44 | 0.48 | 72.2 |
| C45 | 0.45 | 78.9 |
| C47 | 0.25 | 87.0 |

5. Experimental conclusion: The conjugates of the present disclosure can significantly reduce INHBE mRNA levels in PHHs.

### Test Example 3: Mouse hINHBE HDI model

### 1. Experimental principle:

The *in vivo* targeting activity and inhibitory effect of the test compounds on the target gene were evaluated using a mouse model established by hydrodynamic tail vein injection of pcDNA-INHBE plasmid.

### 2. Experimental materials:

pcDNA-INHBE plasmid; female BALB/c mice; DPBS (Dulbecco's phosphate-buffered saline); conjugates of the present disclosure.

### 3. Experimental procedure:

Female BALB/c mice aged 6 to 8 weeks were obtained and underwent a one-week acclimatization and quarantine period upon arrival at the animal facility.

On Day 0, the mice were randomly divided into groups based on their body weight, with 4 to 6 mice per group. After grouping, all mice received a single subcutaneous injection at a volume of 10 mL/kg. Group 1 received DPBS, while the remaining groups received the conjugates of the present disclosure.

On Day 7 after dosing, all mice received hydrodynamic tail vein injection of pcDNA-INHBE plasmid within 5 seconds at a volume equivalent to 8% of their body weight (injection volume (mL) = body weight (g) × 8%), with each mouse receiving 10 µg of plasmid DNA.

On Day 8 after dosing, all mice were euthanized by CO₂ inhalation, and two liver samples were collected from each mouse. Liver samples were treated with RNAlater at 4°C overnight, after which the RNAlater was removed, and the samples were stored at -80°C for subsequent analysis of INHBE gene expression.

4. The experimental results are shown in Tables 7 to 9.

**Table 7: Inhibition of hepatic INHBE mRNA expression by conjugates C1 and C23 of the present disclosure in mice**

| Animal No. | Group 1 | Group 2 | Group 3 |
|---|---|---|---|
| | PBS (%) | Conjugate C1, 5 mg/kg (remaining mRNA, %) | Conjugate C23, 5 mg/kg (remaining mRNA, %) |
| 1 | 97 | 13 | 27 |
| 2 | 83 | 11 | 22 |
| 3 | 90 | 16 | 16 |
| 4 | 135 | 15 | 25 |
| 5 | 95 | / | / |
| Mean | 100 | 13.8 | 22.5 |

| | | | |
|---|---|---|---|
| "/" indicates no data; p < 0.05 indicates a statistically significant difference. | | | |

**Table 8: Inhibition of hepatic INHBE mRNA expression in mice by conjugate C25 of the present disclosure**

| Animal No. | Group 1 | Group 2 |
|---|---|---|
| | PBS (%) | Conjugate C25, 5 mg/kg (remaining mRNA, %) |
| 1 | 100 | 8 |
| 2 | 91 | 14 |
| 3 | 101 | 7 |
| 4 | 101 | 6 |
| 5 | 107 | 11 |
| 6 | / | 16 |
| Mean | 100 | 10 |

| | | |
|---|---|---|
| "/" indicates no data; p < 0.05 indicates a statistically significant difference. | | |

**Table 9: Inhibition of hepatic INHBE mRNA expression in mice by conjugates C42-C45 and C47 of the present disclosure**

| Animal No. | Group 5 mg/kg (remaining mRNA, %) | | | | | |
|---|---|---|---|---|---|---|
| | PBS (%) | Conjugate C42 | Conjugate C43 | Conjugate C44 | Conjugate C45 | Conjugate C47 |
| 1 | 107 | 13 | 10 | 15 | 21 | 19 |
| 2 | 137 | 14 | 14 | 19 | 33 | 20 |
| 3 | 90 | 14 | 10 | 19 | 30 | 16 |
| 4 | 67 | 16 | 11 | 16 | 19 | 17 |
| Mean | 100 | 14 | 11 | 18 | 26 | 18 |

| | | | | | | |
|---|---|---|---|---|---|---|
| "/" indicates no data; p < 0.05 indicates a statistically significant difference. | | | | | | |

5. Experimental conclusion: The conjugates of the present disclosure can significantly reduce hepatic INHBE mRNA levels in mice.

### Test Example 4: Mouse hINHBE HDI model

### 1. Experimental principle:

The *in vivo* targeting activity and inhibitory effect of the test compounds on the target gene were evaluated using a mouse model established by hydrodynamic tail vein injection of pcDNA-INHBE plasmid.

### 2. Experimental materials:

pcDNA-INHBE plasmid; female BALB/c mice; DPBS (Dulbecco's phosphate-buffered saline); conjugates of the present disclosure.

### 3. Experimental procedure:

Female BALB/c mice aged 6 to 8 weeks were obtained and underwent a one-week acclimatization and quarantine period upon arrival at the animal facility.

On Day 0, the mice were randomly divided into groups based on their body weight, with 6 to 7 mice per group. After grouping, all mice received a single subcutaneous injection at a volume of 10 mL/kg. Group 1 received DPBS; Group 2 received conjugate C43; and Group 3 received conjugate C47.

On Day 14 after dosing, all mice received hydrodynamic tail vein injection of pcDNA-INHBE plasmid within 5 seconds at a volume equivalent to 8% of their body weight (injection volume (mL) = body weight (g) × 8%), with each mouse receiving 10 µg of plasmid DNA.

On Day 15 after dosing, all mice were euthanized by CO₂ inhalation, and two liver samples were collected from each mouse. Liver samples were treated with RNAlater at 4°C overnight, after which the RNAlater was removed, and the samples were stored at -80°C for subsequent analysis of INHBE gene expression.

4. The experimental results are shown in Table 10.

**Table 10: Relative expression of hepatic INHBE mRNA of mice in the treatment groups compared to those in the PBS group**

| Animal No. | Group 1 | Group 2 | Group 3 |
|---|---|---|---|
| | PBS (%) | Conjugate C43, 5 mg/kg (remaining mRNA, %) | Conjugate C47, 5 mg/kg (remaining mRNA, %) |
| 1 | 89 | 4 | 13 |
| 2 | 143 | 3 | 15 |
| 3 | 105 | 6 | 18 |
| 4 | 83 | 4 | 8 |
| 5 | 83 | 6 | 14 |
| 6 | 99 | 5 | 13 |
| 7 | 99 | / | / |
| Mean | 100 | 5 | 14 |

| | | | |
|---|---|---|---|
| "/" indicates no data; p < 0.05 indicates a statistically significant difference. | | | |

5. Experimental conclusion: The conjugates of the present disclosure can significantly reduce hepatic INHBE mRNA levels in mice, exhibiting long-lasting efficacy.

### Test Example 5: In vivo INHBE mRNA knockdown study in non-human primates (cynomolgus monkeys)

### 1. Experimental principle:

The degree of reduction in hepatic INHBE mRNA in cynomolgus monkeys induced by the test compounds was evaluated using a non-human primate (cynomolgus monkey) model, which exhibits high homology to the human INHBE gene.

2. Experimental materials: cynomolgus monkeys; 1× PBS (phosphate-buffered saline); conjugates of the present disclosure.

### 3. Experimental procedure:

Four cynomolgus monkeys weighing 2 to 4 kg were obtained and underwent a one-week acclimatization and quarantine period upon arrival at the animal facility.

Three days prior to dosing, the cynomolgus monkeys were randomly divided into groups based on their body weight, with two animals per group.

On Day 1, the conjugates of the present disclosure were subcutaneously injected at a dose of 3 mpk. Blood and liver tissue samples were collected on Days 15 and 29 after dosing. Hepatic INHBE mRNA levels in cynomolgus monkeys were measured using the qPCR method described above. By comparing the hepatic INHBE mRNA levels after dosing with those prior to dosing, the percentage of INHBE mRNA knockdown induced by the conjugates in each group was determined.

4. The experimental results are shown in Table 11.

**Table 11: Inhibition of hepatic INHBE mRNA expression in cynomolgus monkeys by conjugates of the present disclosure**

| Animal No. | Group 1 | Group 2 |
|---|---|---|
| | Conjugate C43 (remaining mRNA, %) | Conjugate C47 (remaining mRNA, %) |
| Day -3 | 100 | 100 |
| Day 15 | 84 | 48 |
| Day 29 | 51 | 30 |

5. Experimental conclusion: The conjugates of the present disclosure can significantly reduce hepatic INHBE mRNA expression in cynomolgus monkeys, exhibiting good *in vivo* inhibitory activity.

### Test Example 6: Liver homogenate stability assay

### 1. Experimental principle:

The test compounds were incubated with liver homogenates from mice, rats, cynomolgus monkeys, and humans to evaluate the stability of these compounds in the liver homogenates.

2. Experimental materials: liver homogenates from mice, rats, cynomolgus monkeys, and humans; conjugates of the present disclosure.

### 3. Experimental procedure:

3.1 A 250 µL working solution of each test compound was prepared at a concentration of 20 µM.

3.2 Frozen liver homogenates were thawed in a water bath at 37°C.

3.3 190 µL of liver homogenate was added to each well of a 96-well plate.

3.4 10 µL of the working solution was added to each well containing 190 µL of liver homogenate.

3.5 The 96-well plate containing the liver homogenate and test compound was incubated at 37°C for up to 48 hours.

3.6 Incubation was terminated at different time points. The test compound was extracted. The concentrations of both the sense and antisense strands of the test compound were determined by LC-MS/MS. The percentage of the remaining compound was then calculated.

4. The experimental results are shown in Table 12. All values are expressed as percentages.

**Table 12: Remaining levels of sense and antisense strands of test compounds following incubation in liver homogenates from different species**

| Incubation time | C47 antisense strand | | | | C47 sense strand | | | |
|---|---|---|---|---|---|---|---|---|
| | Mouse | Rat | Cynomolgus monkey | Human | Mouse | Rat | Cynomolgus monkey | Human |
| 0 | 100.0 | 100.0 | 100.0 | 100.0 | 100.0 | 100.0 | 100.0 | 100.0 |
| 2 | 99.4 | 96.6 | 92.2 | 116.3 | 78.0 | 54.5 | 12.7 | 95.2 |
| 4 | 101.0 | 99.6 | 94.2 | 102.9 | 51.6 | 30.4 | 2.8 | 81.0 |
| 6 | 103.9 | 103.2 | 97.4 | 109.3 | 33.6 | 17.4 | 0.8 | 77.6 |
| 24 | 103.3 | 109.3 | 94.1 | 94.4 | 1.2 | 1.5 | 0.0 | 24.9 |
| 48 | 94.9 | 101.6 | 93.5 | 96.9 | 0.0 | 0.0 | 0.0 | 10.2 |

5. Experimental conclusion: The antisense strand of the conjugate of the present disclosure exhibits good stability in liver homogenates from mice, rats, cynomolgus monkeys, and humans, indicating that the antisense strand can persist *in vivo* for an extended period and thereby exert sustained pharmacological activity.

### Test Example 7: In vitro RNA sequencing analysis

### 1. Experimental principle:

The conjugates of the present disclosure were incubated with primary human hepatocytes (PHHs) for 48 hours. The cells were then lysed to obtain cell lysates, from which total RNA was extracted using a standard extraction method. Following standard procedures, sequencing was performed to evaluate the gene regulatory effects of the conjugates of the present disclosure in PHHs and to assess their off-target potential.

2. Experimental materials: conjugates of the present disclosure; PHHs.

### 3. Experimental methods

3.1 The test conjugates were administered to PHHs via free uptake (48 hours).
1) Preparation of plating medium: InvitroGRO CP Medium containing 10% FBS and 1% penicillin-streptomycin.
2) Cryopreserved PHHs were removed from liquid nitrogen storage and immediately thawed in a water bath at 37°C until a small ice crystal remained in the tube.
3) 2 mL of thawed PHHs was added to 18 mL of plating medium and mixed gently by inversion.
4) 100 µL of diluted conjugate solution was added to each well of a collagen-coated 12-well culture plate, followed by addition of 900 µL of cell suspension per well.
5) After plating, the cells were incubated at 37°C with 5% CO₂ for 48 hours.

### 3.2 Collection of cell lysates

1) After 48 hours of cell culture, the culture plate was removed and cell morphology was examined under a microscope.
2) The culture medium was removed, and each well was washed with 500 µL of PBS. Subsequently, 400 µL of TRIzol (RNA extraction reagent) was added to each well of the 12-well plate.
3) Complete cell lysis was confirmed under a microscope. Replicate lysates were pooled, transferred to a 1.5 mL centrifuge tube, and store at -80°C.

3.3 Sequencing: Total RNA was extracted from the TRIzol lysates and used for library preparation. The resulting libraries were subjected to high-throughput sequencing.

4. The experimental results are shown in Table 13.

**Table 13: Regulation of gene expression in PHHs by conjugates of the present disclosure**

| Comparison | Total number of differentially expressed genes | Upregulated genes | Downregulated genes | Major downregulated gene | Threshold |
|---|---|---|---|---|---|
| C47 vs. PBS | 12 | 7 | 5 | INHBE | padj ≤ 0.05; \|log₂FoldChange\| ≥ 1.0 |
| C43 vs. PBS | 1 | 0 | 1 | INHBE | padj ≤ 0.05; \|log₂FoldChange\| ≥ 1.0 |

5. Experimental conclusion: The conjugates of the present disclosure exhibit a low risk of off-target effects and high target specificity in *in vitro* RNA sequencing analysis.

## Claims

1. A double-stranded siRNA or a pharmaceutically acceptable salt thereof, wherein the double-stranded siRNA comprises a sense strand and an antisense strand capable of forming a duplex region; the sense strand comprises at least 16 consecutive nucleotides from any one of the sequences set forth in SEQ ID NOs: 1-80 and SEQ ID NOs: 161-188; the antisense strand comprises at least 16 consecutive nucleotides from any one of the sequences set forth in SEQ ID NOs: 81-160 and SEQ ID NOs: 189-230;
the nucleotides comprised in the sense strand and the nucleotides comprised in the antisense strand are modified or unmodified nucleotides.

2. The double-stranded siRNA or the pharmaceutically acceptable salt thereof according to claim 1, wherein the sense strand comprises the nucleotides at positions 1-17, 1-18, 1-19, 1-20, 1-21, 2-17, 2-18, 2-19, 2-20, 2-21, 3-18, 3-19, 3-20, 3-21, 4-19, 4-20, 4-21, or 5-21 from the 5' end of any one of the sequences set forth in SEQ ID NOs: 1-80 and SEQ ID NOs: 161-188; preferably, the sense strand comprises the nucleotides at positions 1-17, 1-18, 1-19, 2-17, 2-18, 2-19, 2-20, 3-18, 3-19, 3-20, 4-19, 4-20, 4-21, or 5-21 from the 5' end of any one of the sequences set forth in SEQ ID NOs: 1-80 and SEQ ID NOs: 161-188.

3. The double-stranded siRNA or the pharmaceutically acceptable salt thereof according to claim 1, wherein the antisense strand comprises the nucleotides at positions 1-17, 1-18, 1-19, 1-20, 1-21, 1-22, 1-23, 2-18, 2-19, 2-20, 2-21, 2-22, 2-23, 3-19, 3-20, 3-21, 3-22, 3-23, 4-20, 4-21, 4-22, 4-23, 5-21, 5-22, 5-23, 6-22, 6-23, or 7-23 from the 5' end of any one of the sequences set forth in SEQ ID NOs: 81-160 and SEQ ID NOs: 189-230; preferably, the antisense strand comprises the nucleotides at positions 1-19, 1-20, 1-21, 1-22, 1-23, 2-18, 2-19, 2-20, 2-21, 3-20, or 3-21 from the 5' end of any one of the sequences set forth in SEQ ID NOs: 81-160 and SEQ ID NOs: 189-230.

4. The double-stranded siRNA or the pharmaceutically acceptable salt thereof according to any one of claims 1 to 3, wherein at least one nucleotide residue on the sense strand, the antisense strand, or both the sense strand and the antisense strand is independently and optionally replaced by dR, R, or Z, wherein the dR is 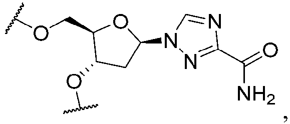 the R is 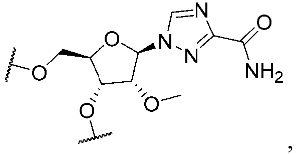 and the Z is

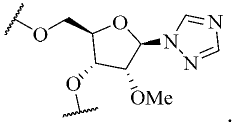

5. The double-stranded siRNA or the pharmaceutically acceptable salt thereof according to claim 4, wherein 1 to 4 nucleotide residues at positions 2, 5-7, 12, and 14-18 from the 5' end of the antisense strand are independently and optionally replaced by dR, R, or Z.

6. The double-stranded siRNA or the pharmaceutically acceptable salt thereof according to claim 4, wherein 1 to 4 nucleotides at positions 2, 5-7, 12, and 14-18 from the 5' end of the antisense strand are independently and optionally 2'-deoxynucleotides.

7. The double-stranded siRNA or the pharmaceutically acceptable salt thereof according to claim 1, wherein the first nucleoside at the 5' end of the antisense strand may be replaced by a vinylphosphonate-containing nucleoside analog.

8. The double-stranded siRNA or the pharmaceutically acceptable salt thereof according to claim 7, wherein the vinylphosphonate-containing nucleoside analog is selected from the following structural moieties:

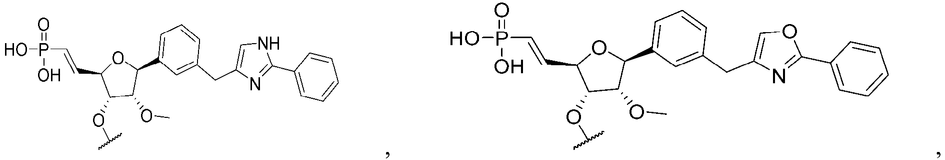

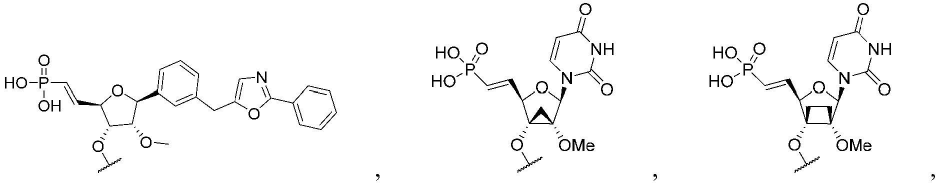

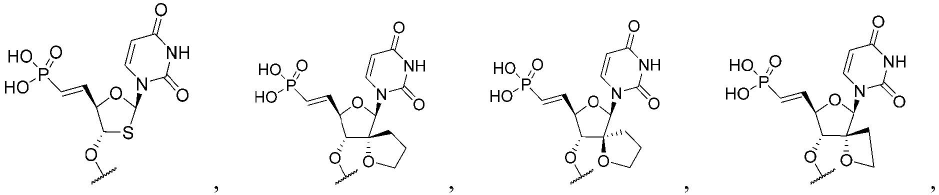

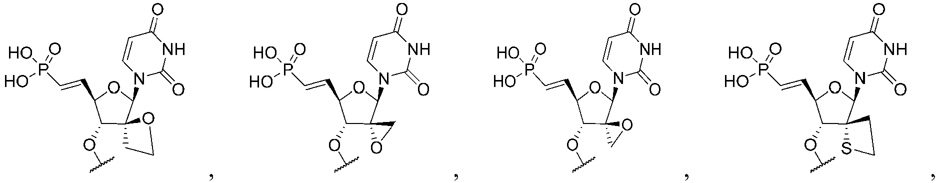

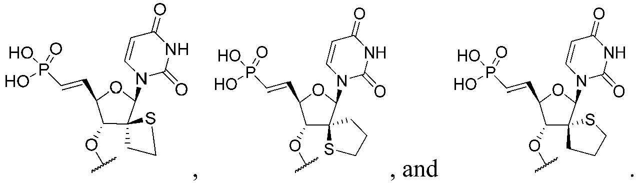

9. A double-stranded siRNA or a pharmaceutically acceptable salt thereof, comprising a sense strand and an antisense strand capable of forming a duplex region, wherein the combination of the nucleotide sequence of the sense strand and the nucleotide sequence of the antisense strand is selected from any one of the nucleotide sequence combinations set forth in S83-S129.

10. A conjugate or a pharmaceutically acceptable salt thereof, comprising the double-stranded siRNA according to any one of claims 1 to 9 and a pharmaceutically acceptable conjugate moiety.

11. The conjugate or the pharmaceutically acceptable salt thereof according to claim 10, wherein the conjugate moiety is selected from the following structures: 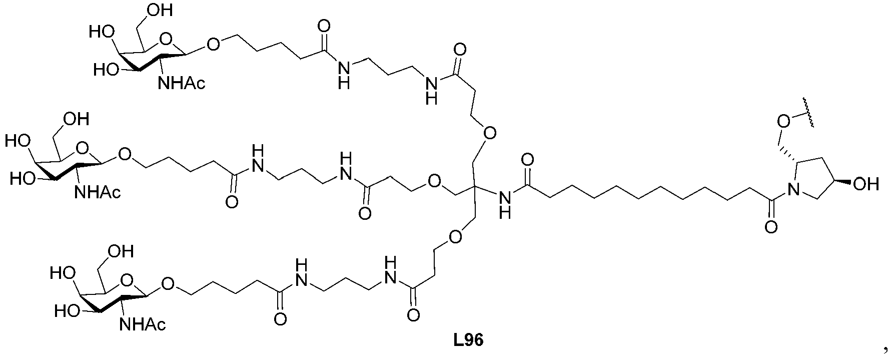 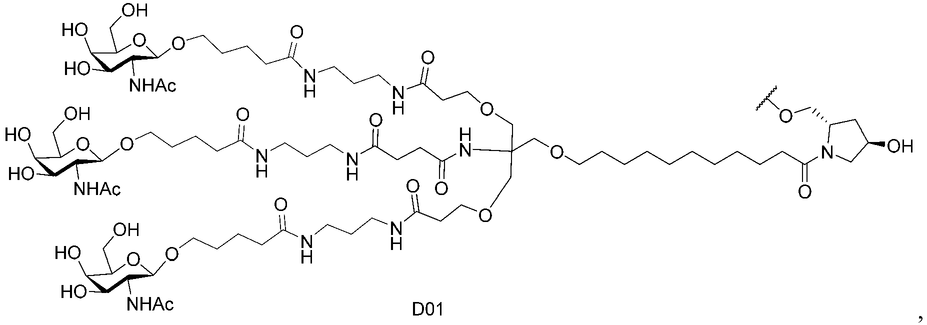 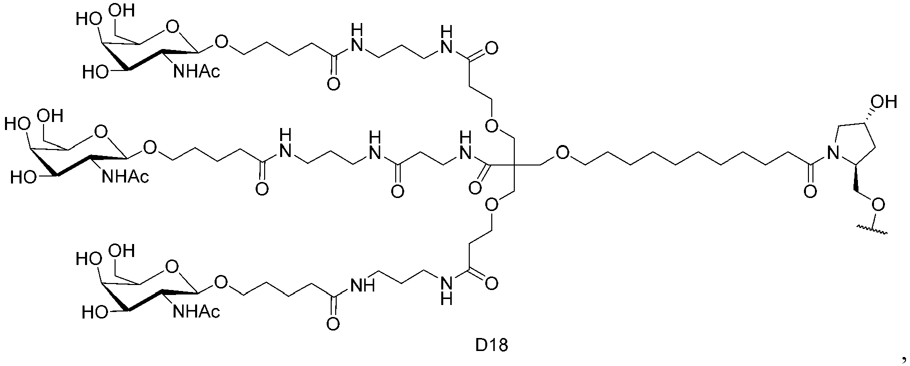 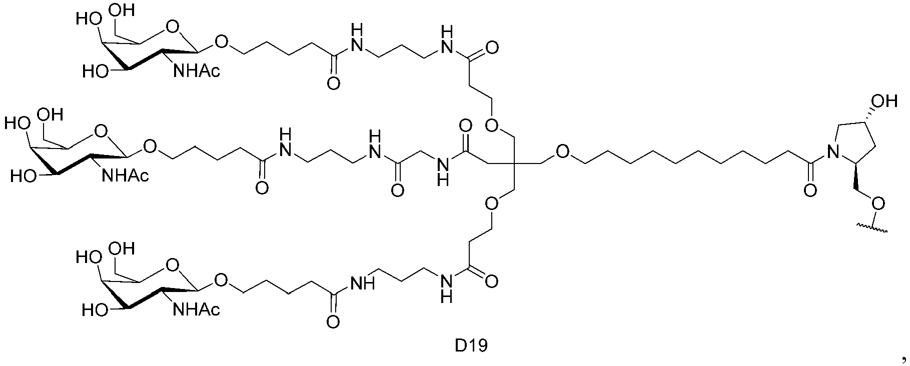 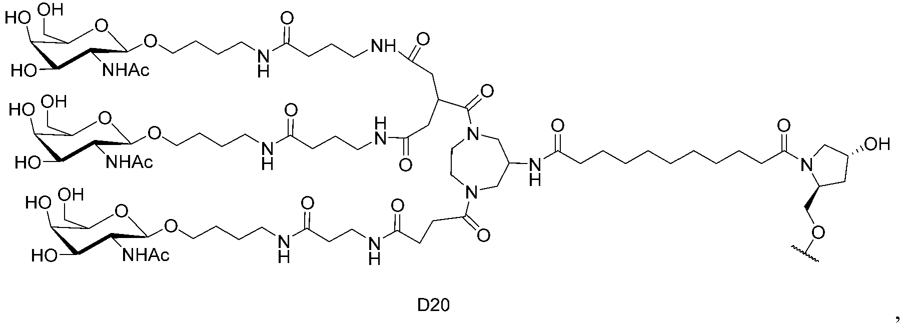 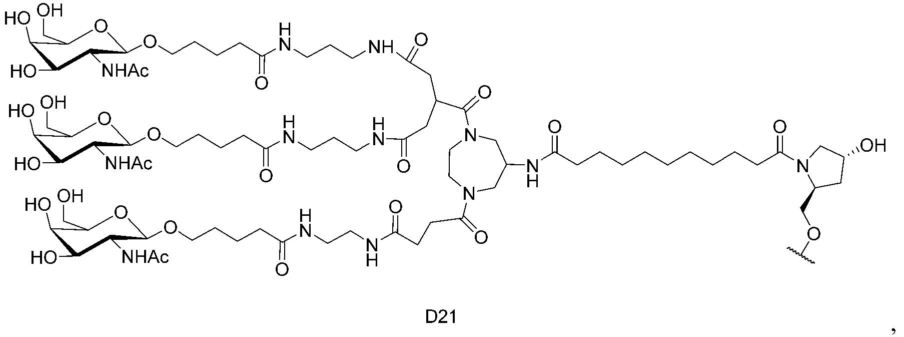 and

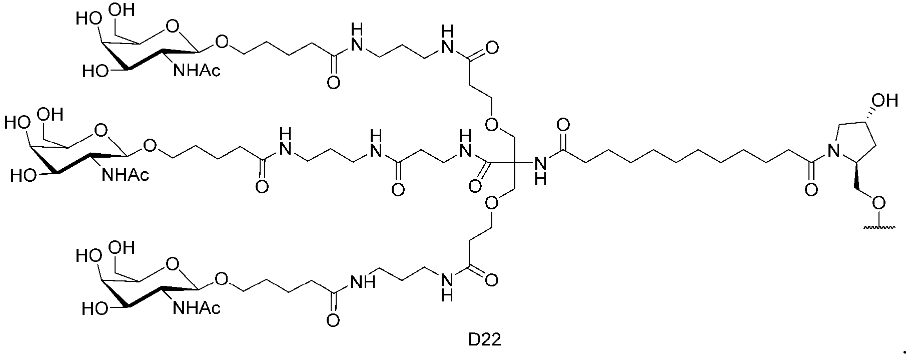

12. The conjugate or the pharmaceutically acceptable salt thereof according to claim 11, wherein the conjugate is selected from C1-C47.

13. The conjugate or the pharmaceutically acceptable salt thereof according to claim 10, wherein the conjugate is represented by formula (I): 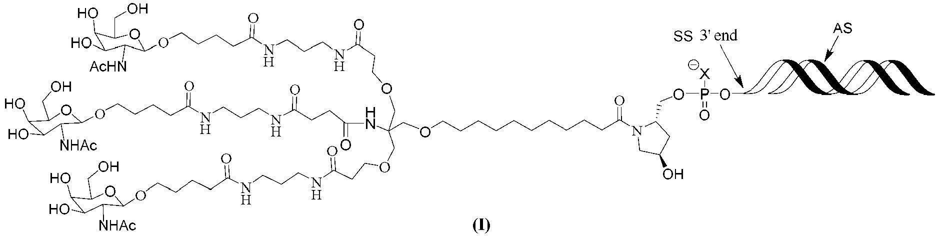 wherein X is O or S; SS represents the sense strand and AS represents the antisense strand; the sense strand and the antisense strand are selected from any one of the following combinations (1) to (9):
(1) the sense strand is set forth in SEQ ID NO: 161, and the antisense strand is set forth in SEQ ID NO: 189;
(2) the sense strand is set forth in SEQ ID NO: 161, and the antisense strand is set forth in SEQ ID NO: 210;
(3) the sense strand is set forth in SEQ ID NO: 186, and the antisense strand is set forth in SEQ ID NO: 226;
(4) the sense strand is set forth in SEQ ID NO: 188, and the antisense strand is set forth in SEQ ID NO: 225;
(5) the sense strand is set forth in SEQ ID NO: 164, and the antisense strand is set forth in SEQ ID NO: 192;
(6) the sense strand is set forth in SEQ ID NO: 171, and the antisense strand is set forth in SEQ ID NO: 199;
(7) the sense strand is set forth in SEQ ID NO: 161, and the antisense strand is set forth in SEQ ID NO: 202;
(8) the sense strand is set forth in SEQ ID NO: 185, and the antisense strand is set forth in SEQ ID NO: 220;
(9) the sense strand is set forth in SEQ ID NO: 188, and the antisense strand is set forth in SEQ ID NO: 228.

14. Use of the double-stranded siRNA or the pharmaceutically acceptable salt thereof according to claims 1 to 9, and the conjugate or the pharmaceutically acceptable salt thereof according to claims 10 to 13, in the manufacture of a medicament for treating a disease associated with INHBE gene expression.

15. The use according to claim 14, wherein the disease associated with INHBE gene expression is a metabolic disease caused by abnormal body fat distribution.

16. The use according to claim 15, wherein the metabolic disease caused by abnormal body fat distribution is selected from type 2 diabetes, obesity, abdominal obesity, coronary heart disease, non-alcoholic fatty liver disease, non-alcoholic steatohepatitis, hyperlipidemia, dyslipidemia, and atherosclerosis.
